# EUROPEAN PATENT SPECIFICATION

(11) **EP 1 335 722 B1**
(45) Date of publication and mention of the grant of the patent: **30.08.2006**
(21) Application number: 01997304.9
(22) Date of filing: 16.11.2001
(51) Int. Cl.: A61K 31/404, A61P 25/24

(54) **INDOLYL-SULFONYL- COMPOUNDS USEFUL IN THE TREATMENT OF CNS DISORDERS**
INDOLY-LSULPHONYL-VERBINDUNGEN ZUR BEHANDLUNG VON STÖRUNGEN DES ZNS
DERIVÉS INDOLE-SULFONIQUES POUR LE TRAITEMENT DE TROUBLES DU SNC

(30) Priority: 24.11.2000 GB 0028708; 04.06.2001 GB 0113517
(43) Date of publication of application: 20.08.2003
(73) Proprietor: SMITHKLINE BEECHAM PLC, Brentford, Middlesex TW8 9GS (GB)
(72) Inventor: BROMIDGE, Steven Mark, GlaxoSmmithKline, Essex CM 19 5AW (GB)
(74) Representative: Valentine, Jill Barbara
(86) International application number: PCT/EP2001/013411
(87) International publication number: WO 2002/041889

(56) References cited:
- WO-A-02/32863
- WO-A-96/03400
- US-A- 6 100 291
- SAKAMOTO, TAKAO ET AL: "Preparation and palladium-catalyzed arylation of indolylzinc halides" JOURNAL OF THE CHEMICAL SOCIETY, PERKIN TRANSACTIONS 1: ORGANIC AND BIO-ORGANIC CHEMISTRY (1996), (16), 1927-1934 , XP002104812

## Description

This invention relates to novel indole compounds having pharmacological activity, processes for their preparation, to compositions containing them and to their use in the treatment of CNS and other disorders.

WO 98/27081 discloses a series of aryl sulphonamide compounds that are said to be 5-HT₆ receptor antagonists and which are claimed to be useful in the treatment of various CNS disorders. GB-2341549, WO 99/47516 and WO 99/65906 all disclose a series of indole derivatives that are claimed to possess 5-HT₆ receptor affinity. WO 96/03400 (Pfizer Inc) describe the use of 1H-indole, 4-(1-methyl-4-piperidinyl)-1 -(phenylsulfonyl) as an intermediate in the preparation of 4-heterocyclylindole derivatives as serotonin agonists and antagonists. JP 61205256 (Sagami Chemical Research Center) and Hatanaka, N., Watanabe, N. and Matsumoto, M. (1986) Heterocycles 24(7), 1987-1996 describe the use of 1H-indole, 4-(1,3-dithian-2-y))-1-(4-methylphenyl)sulfonyl in the short step synthesis of 4-formylindole and derivatives. JP 03024470 (Sagami Chemical Research Center) and Matsumoto, M., Ishida, Y. and Hatanaka, N. (1986) Heterocycles 24(6), 1667-1674 describe the use of 1 H-indole, 1-[(4-methylphenyl)sulfonyl]-4-(4-morpholinyl) in the one-step synthesis of 4-aminoindoles. WO 02/32863 (Biovitrum AB) describe a series of indole sulfones that are claimed to have affinity for the 5-HT₆ receptor.

A structurally novel class of compounds has now been found which also possess 5-HT₆ receptor affinity. The present invention therefore provides, in a first aspect, a compound of formula (I) or a pharmaceutically acceptable salt thereof: 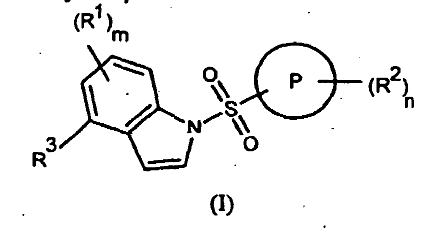 wherein:
P is phenyl, naphthyl or heteroaryl;
R¹ is chlorine, bromine or cyano;
R² is halogen, C₁₋₆alkyl, C₃₋₆cycloalkyl, C₁₋₆alkoxy, C₁₋₆alkylthio, C₁₋₆alkylsulphinyl, C₁₋₆alkylsulphonyl, C₁₋₆alkanoyl, CN, CF₃, OCH₂CF₃, OCF₃, hydroxy, hydroxyC₁₋₆alkyl, hydroxyC₁₋₆alkoxy, C₁₋₆alkoxcarbonyl, C₁₋₆alkoxyC₁₋₆alkoxy, nitro, amino, N(C₁₋₆ alkyl)₂, NHC₁₋₆ alkyl, C₁₋₆alkylamino or diC₁₋₆alkyiamino; or
R² is a group C(O)OR⁴, CONR⁵R⁶ or NR⁵COR⁶ where R⁴ is hydrogen or C₁₋₆alkyl, and R⁵ and R⁶ are independently hydrogen, Chalkyl or R⁵ and R⁶ combine together to form a 5- to 7-membered azacyclic ring optionally containing an additional heteroatom selected from nitrogen, sulphur or oxygen; or
R² is phenyl, naphthyl or heteroaryl optionally substituted by halogen, C₁₋₆alkyl, C₁₋₆alkoxy, C₁₋₆alkanoyl, CN, CF₃, OCF₃, phenyloxy, benzyloxy or C₃₋₆cycloalkyloxy;
R³ is a 5- to 7-membered heterocyclic ring or a bicyclic heterocyclic ring containing 1 to 3 heteroatoms selected from nitrogen, sulphur or oxygen, said rings being optionally substituted by one or more C₁₋₆alkyl groups;
m is 1 or 2; or
n is 0 *-* 5*.*
In one embodiment there is provided a pharmaceutical composition comprising a compound of formula (I) or a pharmaceutically acceptable salt thereof and a pharmaceutically acceptable carrier or excipient.

In one aspect of the present invention R² is not C₁₋₆ alkoxycarbonyl; OCH₂CF₃; N(C₁₋₆ alkyl)₂; NHC₁₋₆ alkyl; a group C(O)OR⁴, CONR⁵R⁶ or NR⁵COR⁶ where R⁴ is hydrogen or C₁₋₆alkyl, and R⁵ and R⁶ are independently hydrogen, C₁₋₆alkyl or R⁵ and R⁶ combine together to form a 5- to 7- membered azacyclic ring optionally containing an additional heteroatom selected from nitrogen, sulphur or oxygen; or
phenyl, naphthyl or heteroaryl optionally substituted by halogen, C₁₋₆alkyl, C₁₋₆alkoxy, C₁₋₆alkanoyl, CN, CF₃, OCF₃, phenyloxy, benzyloxy or C₃₋₆cycloalkyloxy.

In a further aspect of the present invention R² is not OCH₂CF₃; N(C₁₋₆ alkyl)₂; or NHC₁₋₆ alkyl.

Where n > 1, groups R² positioned *ortho* to one another may optionally be fused together to form a ring.
Alkyl groups, whether alone or as part of another group, may be straight chain or branched. The term 'halogen' is used herein to describe, unless otherwise stated, a group selected from fluorine, chlorine, bromine or iodine. Where used herein the term naphthyl is intended, unless otherwise stated, to denote both naphth-1-yl and naphth-2-yl groups.
The term "heteroaryl" is intended to mean an aromatic or a fused bicyclic or tricyclic aromatic ring containing 1 to 3 heteroatoms selected from oxygen, nitrogen and sulphur. Suitable examples of such aromatic rings include thienyl, furyl, pyrrolyl, triazolyl, imidazolyl, oxazolyl, thiazolyl, oxadiazolyl, isothiazolyl, isoxazolyl, thiadiazolyl, pyrazolyl, pyrimidyl, pyridazinyl, pyrazinyl and pyridyl. Suitable examples of such fused aromatic rings include quinolinyl, isoquinolinyl, quinazolinyl, quinoxalinyl, cinnolinyl, indolyl, benzofuranyl, dibenzofuranyl, benzothienyl, benzimidazolyl, benzoxazolyl, benzoxadiazolyl, benzthiadiazolyl, imidazothiazolyl and the like. Heteroaryl groups, as described above, may be linked to the remainder of the molecule via a carbon atom or, when present, a suitable nitrogen atom.
The term "5- to 7-membered heterocyclic ring " is intended to mean a non aromatic ring containing 1 to 3 heteroatoms selected from oxygen, nitrogen and sulphur. Such rings may be partially unsaturated. Suitable examples of 5- to 7-membered heterocyclic rings include piperidinyl, tetrahydropyridinyl, pyrrolidinyl, morpholinyl, azepanyl, diazepanyl and piperazinyl.

A 5- to 7-membered heterocyclic ring, as described above, may be linked to the remainder of the molecule via a carbon atom or a suitable nitrogen atom.
When R³ is a bicyclic heterocyclic ring, representative examples of such groups are:

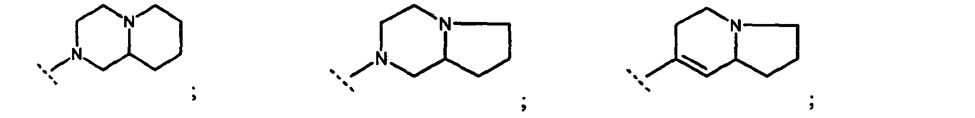

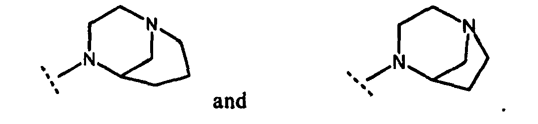

Preferably m = 0, 1, or 2, most preferably 1 or 2.
Preferably n = 0, 1, 2 or 3, most preferably 1.
A preferred substituent for rings within the definition of R³, which can be present on carbon and/or nitrogen atoms, is methyl.
Most preferably R³ is an unsubstituted piperazine or N-methyl piperazine attached to the rest of the molecule via a suitable nitrogen atom. Also most preferably, R³ represents 1,4-diazepanyt linked to the indole moiety via a nitrogen atom optionally substituted by a methyl group. Particularly preferred compounds of formula (I) are those wherein R³ is a piperazinyl or a 1,4-diazepanyl group linked to the indole moiety via a nitrogen atom, both of which may be optionally substituted with a single methyl group. Especially preferred compounds of formula (I) are those wherein R³ is an unsubstituted piperazinyl group linked to the indole moiety via a nitrogen atom.

When m is 2, the groups R¹ may be the same or different. For the avoidance of doubt, the groups R¹ can be substituted at any suitable carbon atom within the indole ring. A particularly preferred R¹ group is chloro in the 5- position of the indole ring. Especially preferred are compounds of formula (I) wherein R¹ represents 5-chloro, 7-chloro or 5,7-dichloro, most especially 5-chloro or 5,7-dichloro.
Preferably, P represents phenyl, naphthyl, benzothiophenyl, thiazolyl, thienyl, oxazolyl, benzthiadiazolyl, benzoxadiazolyl, benzofuranyl, pyrazolyl, pyridinyl, dibenzofuranyl, isoquinolinyl or imidazothiazolyl. Most preferably, P represents phenyl, pyridyl or pyrazolyl, especially phenyl.

When n is other than 0, R² is preferably halogen (particularly chlorine or bromine), a C₁₋₆alkyl group (particularly methyl), CF₃, cyano, C₁₋₆alkoxy group (particularly methoxy) or heteroaryl. Also preferably, R² represents fluorine, iodine, ethyl, propyl, butyl, C(CH₃)₃, OCF₃, NR⁵COR⁶ (particularly NHCOCH₃), NO₂, COOR⁴ (particularly COOH and COOCH₃), C₁₋₆ alkanoyl (particularly COCH₃), heteroaryl (particularly pyridyl and oxazolyl), O(CH₂)₃CH₃, phenyl, N(CH₃)₂ or OCH₂CF₃. Most preferably, R² is chlorine, bromine, methyl, OCF₃ or cyano, especially chlorine. When n is 2, 3 , 4 or 5 the groups R² may be the same or different. R² is most preferably in the 3 position.

Preferred compounds according to the invention include examples E1, E74-78, E132, E135-162, E164, E166, E169-170 and E175 as shown below or a pharmaceutically acceptable salt thereof.

More preferred compounds according to the invention include: 5-Chloro-1-(naphthalene-2-sulfonyl)-4-piperazin-1-yl-1*H*-indole trifluoroacetic acid salt (E77); 5-Chloro-1-(3,5-dichloro-2-methoxy benzenesulfonyl)-4-piperazin-1-yl-1*H*-indole trifluoroacetic acid salt (E78);
5-Chloro-1-(3-chlorobenzenesulfonyl)-4-piperazin-1-yl-1H-indole hydrochloride (E132); 5-Chloro-1-(pyridine-2-sulfonyl)-4-piperazin-1-yl-1*H*-indole hydrochloride (E149); 7-Chloro-1-(3,5-dichlorobenzenesulfonyl)-4-piperazin-1-yl-1*H*-indole hydrochloride (E153); 1-(3-Chlorobenzenesulfonyl)-5,7-dichloro-4-piperazin-1-yl-1*H*-indole hydrochloride (E159); and 7-Chloro-1-(3-chlorobenzenesulfonyl)-4-[1,4]diazepan-1-yl-1*H*-indole hydrochloride (E175) or a pharmaceutically acceptable salt thereof.

The compounds of formula (I) can form acid addition salts thereof. It will be appreciated that for use in medicine the salts of the compounds of formula (I) should be pharmaceutically acceptable. Suitable pharmaceutically acceptable salts will be apparent to those skilled in the art and include those described in J. Pharm. Sci., 1977, 66, 1-19, such as acid addition salts formed with inorganic acids e.g. hydrochloric, hydrobromic, sulfuric, nitric or phosphoric acid; and organic acids e.g. succinic, maleic, acetic, fumaric, citric, tartaric, benzoic, p-toluenesulfonic, methanesulfonic, trifluoroacetic or naphthalenesulfonic acid. The present invention includes within its scope all possible stoichiometric and non-stoichiometric forms.
The compounds of formula (I) may be prepared in crystalline or non-crystalline form, and, if crystalline, may optionally be hydrated or solvated. This invention includes within its scope stoichiometric solvates eg. hydrates as well as compounds containing variable amounts of solvent eg. water.
Certain compounds of formula (I) are capable of existing in stereoisomeric forms (e.g. diastereomers and enantiomers) and the invention extends to each of these stereoisomeric forms and to mixtures thereof including racemates. The different stereoisomeric forms may be separated one from the other by the usual methods, or any given isomer may be obtained by stereospecific or asymmetric synthesis. The invention also extends to any tautomeric forms and mixtures thereof.

The present invention also provides a process for the preparation of a compound of formula (IA) or a pharmaceutically acceptable salt thereof, which process comprises
(a) the coupling of a compound of formula (II): 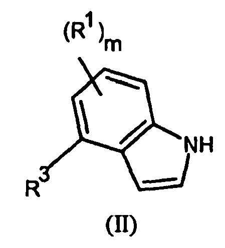 in which R¹, R³ and m are as defined in formula (I) or protected derivatives thereof with a compound of formula (III): 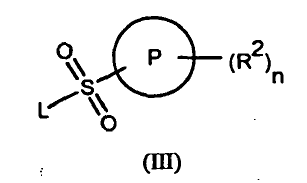 in which P, R² and n are as defined in formula (I) or a protected derivative thereof and L is a leaving group and optionally thereafter:
   - removing any protecting groups,
   - forming a pharmaceutically acceptable salt.

   The present invention also provides further process for the preparation of a compound of formula (IA) or a pharmaceutically acceptable salt thereof, which process comprises:
(b) preparing a compound of formula (IA) wherein R³ represents an optionally substituted piperazinyl or 1,4-diazepanyl group linked to the indole moiety via a nitrogen atom which comprises reacting a compound of formula (IV) 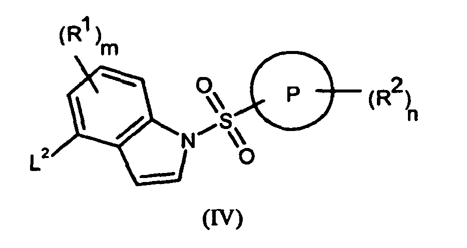 wherein R¹, R², P, m and n are as defined in formula (IA) or a protected derivative thereof, and L² represents a suitable leaving group (eg. a halogen atom such as bromine or a trifluoromethylsulfonyloxy or nonafluorobutylsulfonyloxy group), with a compound of R^{3'}-H, wherein R^{3'} represents an optionally protected and/or substituted piperazinyl or 1,4-diazepanyl group, and optionally thereafter:
   - removing any protecting groups,
   - forming a pharmaceutically acceptable salt; or
(c) deprotecting a compound of formula (IA) which is protected; or
(d) interconversion of a compound of formula (IA) to other compounds of formula (IA).

In process (a), suitable leaving groups include halogen, in particular chloro. The reaction of compounds of formulae (II) and (III) may be carried out by mixing the two reagents together, optionally under phase-transfer conditions, in a mixture of an inert organic solvent such as tetrahydrofuran with an aqueous base such as sodium hydroxide with the addition of a suitable phase-transfer catalyst such as tetrabutylammonium hydroxide. Alternatively in this case, the reaction of compounds of formulae (II) and (III) may be carried out by treating a compound of formula (II) with a suitable base such as sodium hydride, sodium hexamethyldisilazane (NaHMDS) or a suitable strong organic base such as DBU or BEMP in an inert solvent such as tetrahydrofuran or N,N-dimethylformamide to form the anion of (II) and then treating this with a compound of formula (III) in an inert solvent.
Process (b) typically comprises the use of a suitable base such as cesium carbonate, a suitable salt such as palladium salt (eg. palladium acetate), a suitable ligand such as BINAP and a suitable solvent such as 1,4-dioxan.
It will be appreciated by those skilled in the art that it may be necessary to protect certain reactive substituents during some of the above procedures.
In process (c), standard protection and deprotection techniques, such as those described in Greene T.W.'Protective groups in organic synthesis', New York, Wiley (1981), can be used. For example, primary amines can be protected as phthalimide, benzyl, benzyloxycarbonyl or trityl derivatives. Carboxylic acid groups can be protected as esters. Aldehyde or ketone groups can be protected as acetals, ketals, thioacetals or thioketals. Deprotection of such groups is achieved using conventional procedures well known in the art.
Process (d) may be performed using conventional interconversion procedures such as epimerisation, oxidation, reduction, alkylation, nucleophilic or electrophilic aromatic substitution, ester hydrolysis or amide bond formation. For example, interconversion of the groups R¹ and R² or interconversion of one salt of formula (I) to another salt of formula (I), eg. preparing the hydrochloric acid salt from the trifluoroacetic acid salt by treating with excess hydrochloric acid followed by evaporation.
Compounds of formulae (II) and (III) are commercially available, may be prepared using procedures described herein or by analogous methods thereto or according to known methods.
For example, compounds of formula (II) may be prepared from the corresponding protected indole having a leaving group in the 4-position (eg. a halogen atom such as bromine or a trifluoromethylsulfonyloxy or nonafluorobutylsulfonyloxy group) by reaction with a compound of formula R^{3'}-H where R^{3'} represents an N containing heterocycle such as optionally substituted or protected piperazinyl or 1,4-diazepanyl.
Compounds of formula (IV) may be prepared by analogous methods to those described for process (a) above.
Pharmaceutically acceptable salts may be prepared conventionally by reaction with the appropriate acid or acid derivative.

Compounds of formula (I) and their pharmaceutically acceptable salts have 5-HT₆ receptor activity and are believed to be of potential use in the treatment of certain CNS disorders such as anxiety, depression, epilepsy, obsessive compulsive disorders, migraine, cognitive memory disorders (e.g. Alzheimers disease, age related cognitive decline and mild cognitive impairment), Parkinsons Disease, ADHD (Attention Deficit Disorder/Hyperactivity Syndrome), sleep disorders (including disturbances of Circadian rhythm), feeding disorders such as anorexia and bulimia, panic attacks, withdrawal from drug abuse such as cocaine, ethanol, nicotine and benzodiazepines, schizophrenia, and also disorders associated with spinal trauma and/or head injury such as hydrocephalus. Compounds of the invention are also expected to be of use in the treatment of certain GI (gastrointestinal) disorders such as IBS (Irritable Bowel Syndrome).
Thus there is also provided a compound of formula (I) or a pharmaceutically acceptable salt thereof, for use as a therapeutic substance, in particular in the treatment or prophylaxis of depression, anxiety and cognitive memory disorders. Also in particular there is provided a compound of formula (I) or a pharmaceutically acceptable salt thereof, for use in the treatment of Alzheimers disease, age related cognitive decline, mild cognitive impairment, ADHD and schizophrenia.
There is further provided a method of treatment or prophylaxis of the above disorders, in mammals including humans, which comprises administering to the sufferer a therapeutically effective amount of a compound of formula (1) or a pharmaceutically acceptable salt thereof.
In another aspect, there is provided the use of a compound of formula (I) or a pharmaceutically acceptable salt thereof in the manufacture of a medicament for use in the treatment or prophylaxis of the above disorders.
In order to use the compounds of formula (I) in therapy, they will normally be formulated into a pharmaceutical composition in accordance with standard pharmaceutical practice.
A pharmaceutical composition of the invention, which may be prepared by admixture, suitably at ambient temperature and atmospheric pressure, is usually adapted for oral, parenteral or rectal administration and, as such, may be in the form of tablets, capsules, oral liquid preparations, powders, granules, lozenges, reconstitutable powders, injectable or infusable solutions or suspensions or suppositories. Orally administrable compositions are generally preferred.
Tablets and capsules for oral administration may be in unit dose form, and may contain conventional excipients, such as binding agents, fillers, tabletting lubricants, disintegrants and acceptable wetting agents. The tablets may be coated according to methods well known in normal pharmaceutical practice.
Oral liquid preparations may be in the form of, for example, aqueous or oily suspension, solutions, emulsions, syrups or elixirs, or may be in the form of a dry product for reconstitution with water or other suitable vehicle before use. Such liquid preparations may contain conventional additives such as suspending agents, emulsifying agents, non-aqueous vehicles (which may include edible oils), preservatives, and, if desired, conventional flavourings or colourants.
For parenteral administration, fluid unit dosage forms are prepared utilising a compound of the invention or pharmaceutically acceptable salt thereof and a sterile vehicle. The compound, depending on the vehicle and concentration used, can be either suspended or dissolved in the vehicle. In preparing solutions, the compound can be dissolved for injection and filter sterilised before filling into a suitable vial or ampoule and sealing. Advantageously, adjuvants such as a local anaesthetic, preservatives and buffering agents are dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilization cannot be accomplished by filtration. The compound can be sterilised by exposure to ethylene oxide before suspension in a sterile vehicle. Advantageously, a surfactant or wetting agent is included in the composition to facilitate uniform distribution of the compound.
The composition may contain from 0.1% to 99% by weight, preferably from 10 to 60% by weight, of the active material, depending on the method of administration.
The dose of the compound used in the treatment of the aforementioned disorders will vary in the usual way with the seriousness of the disorders, the weight of the sufferer, and other similar factors. However, as a general guide suitable unit doses may be 0.05 to 1000 mg, more suitably 0.05 to 20.0 mg, for example 0.2 to 5 mg; and such unit doses may be administered more than once a day, for example two or three times a day, so that the total daily dosage is in the range of about 0.5 to 100 mg; and such therapy may extend for a number of weeks or months.

The following Descriptions and Examples illustrate the preparation of compounds of the invention.

### Description 1

### 4-(4-Methyl-piperazin-1-yl)-1H indole (D1)

4-Aminoindole (5.0 g, 37.8 mmol) was dissolved in *n*-butanol (100 ml) followed by addition of sodium carbonate (16.0 g, 0.15 mol) and methchlorethamine hydrochloride (8.73 g, 45.4 mmol). The resulting suspension was stirred at room temperature for 48 hours. The solvent was then removed *in vacuo,* the residue taken up in dichloromethane (200 ml) and washed with saturated sodium hydrogencarbonate solution (2x100 ml). The organic phase was dried (MgSO₄) filtered and solvent evaporated. The crude was purified by column chromatography on silica, eluting with a methanol/dichloromethane gradient to afford the title compound (D1) (4.82 g, 59%), δH (CDCl₃)/ppm 2.40 (3H, s), 2.69 (4H, br s), 3.29 (4H, br s), 6.55 (1H, t, J = 4.0 Hz), 6.60 (1H, d, J = 7.2 Hz), 7.05 (1H, d, J = 8.6 Hz), 7.10 (1H, d, J = 7.6 Hz), 7.14 (1H, t, J = 3.2 Hz). MS: m/z (MH⁺) 216.

### Description 2

### 4-(4-Benzyl-piperazin-1-yl)-1-(5-chloro-3-methyl-benzo[b]thiophene-2-sulfonyl)-1H-indole (D2)

A 40 wt % aqueous solution of tetrabutylammonium hydroxide (0.015 ml, 0.022 mmol) was added to a stirred solution of 4-(4-benzyl-piperazin-1-yl)-1*H*-indole (for synthesis see: Mewshaw *et al.* Bioorg. Med. Chem. Lett. 1998, 19.(8), 2675-2680) (102 mg, 0.35 mmol) in tetrahydrofuran (1 ml) at ambient temperature. To this vigorously stirred solution was added a 50 wt % solution of sodium hydroxide (1 ml) in one portion followed, after a 5 minute period, by a solution of 5-chloro-3-methyl-benzo[b]thiophene-2-sulfonyl chloride (98 mg, 0.35 mmol) in tetrahydrofuran (0.5 ml) over 10 minutes. The mixture was stirred vigorously for 3 hours at ambient temperature, then diluted with ethyl acetate (15 ml). The mixture was washed with water (2 x 15 ml) and the organic layer was separated, dried (MgSO₄) and concentrated *in vacuo.* The residue was purified by column chromatography on silica gel eluting with an acetone/toluene gradient to afford the title compound (D2) (82 mg, 44%) as a foam. MS: m/z (MH⁺) 536/538.

### Description 3

### Preparation of 4-nitrophenylchloroformyl bound to Wang resin (D3)

Polystyrene Wang resin (1.95 g, 1.7 mmol/g; bead size 150-300 microns) was suspended in DCM (50 ml) and cooled to 0°C in an ice bath. N-Methylmorpholine (1.1 ml, 9.93 mmol) was added, followed by 4-nitrophenyl chloroformate (2 g, 9.93 mmol) in portions with manual mixing. The mixture was brought to room temperature and shaken for 24 hours under argon. The title resin (D3) was filtered and washed with DCM (100 ml x 5) then dried *in-vacuo* and used in Description 4.

### Description 4

### Preparation of 1-(4-indolyl)piperazin-4-yl bound to Wang resin (D4)

The product from Description 3 (assumed 3.31 mmol) was shaken with DMF (40 ml) for 30 seconds before 4-piperazin-1-yl indole (1.33 g, 6.62 mmol) was added in DMF (10 ml) and the mixture was shaken for 48 hours. The resin was then filtered and washed with DMF (100 ml x 4) and these washings kept to retrieve the excess amine. The resin was then further washed with DMF (100 ml x 2), DCM (100 ml x 5), DCM/MeOH (1:1, 100 ml x 2), MeOH (100 ml x 2), diethyl ether (100 ml x 2) to give the title resin (D4) which was dried *in-vacuo* and used in Description 5.

### Description 5

### General procedure for sulfonation of 1-(4-indolyl)piperazine-4-yl bound Wang resin (D5)

The general coupling procedure was performed by automation on a Myriad Core robotic system. All operations were performed by automation unless stated otherwise.

The resin from Description 4 (80 mg, assumed 0.14 mmol) was weighed into 2 ml Myriad reaction vessels. THF (1 ml) was added and the reactions flushed with argon. Potassium *tert-*butoxide (0.21 ml of 1M solution in THF, 0.21 mmol) in THF (0.7 ml) was added and the mixture was agitated at room temperature for 30 minutes. To each vessel was manually added the appropriate sulfonyl chloride (0.28 mmol) in THF (0.5 ml)(the sulfonyl chlorides used correspond to RSO₂Cl as shown in Table 1) and the mixtures were agitated for 5 hours under argon at room temperature. The resin products were manually filtered and washed successively with DMF/water (9:1, 2 ml x 2), THF (2 ml x3), DCM (2 ml x 3), DCM/MeOH (1:1, 2 ml x 3), MeOH (2 ml x3), DCM (2 ml x3), diethyl ether (2 ml x3) to afford the individual resin-bound 1-sulfonyl indoles (D5).

### Description 6

### 4-(5-Cbloro-1 H-indol-4-yl)-piperazine-l-carboxylic acid tert-butyl ester (D6)

To a stirred solution of 4-(1*H*-indol-4-yl)-piperazine-1-carboxylic acid *tert-butyl* ester (for synthesis see: WO 9967237 (1999)) (0.12 g, 0.4 mmol) in 1,4-dioxane (5 ml) was added N-chlorosuccinimide (53 mg, 0.4 mmol). After stirring for 30 minutes a mixture of water/ethanol (2 ml, 1:1 v/v) was added and the resulting solution concentrated *in vacuo.* DCM (20 ml) was added and the organic phase washed with brine (10 m1), dried (MgSO₄) and filtered. The, filtrate was concentrated *in vacuo* and purified by column chromatography over silica gel, eluting with DCM to afford the title compound (D6) (75 mg, 56%), δH (CDCl₃)/ppm 1.51 (9H, s), 3.32 (4H, br s), 3.62 (4H,'br s), 6.69 (1H, t, J = 2.4 Hz), 7.07 (1H, d, J = 8.8 Hz), 7.16=7.18 (2H, m), 8.20 (1H,br s): MS: m/z (M-H)⁻ 334/336.

### Description 7

### 4-[5-Cbloro-l-(3-cbloro-benzenesulfonyl)-lH-indol-4-yl)-piperazine-l-carboxylic acid tert-butyl ester (D7)

To a stirred suspension of sodium hydride (11 mg, 0.3 mmol, 60% suspension in oil) in DMF (2 ml) was added dropwise 4-(5-chloro-1*H-*indol-4-yl)-piperazine-1-carboxylic acid *tert-butyl* ester (D6) (75 mg, 0.2 mmol) as a solution in DMF (1 ml). After stirring for 20 minutes, a solution of 3-chlorobenzenesulfonyl chloride (70 mg, 0.3 mmol) in DMF (1 ml) was added dropwise and the resulting mixture left to stir for 15 hours. The reaction mixture was then diluted with DCM (20 ml) and washed with water (2x10 ml), brine (10 ml), dried (MgSO₄) and filtered. The filtrate was concentrated *in vacuo* and purified by column chromatography over silica gel, eluting with DCM to afford the title compound (D7) (88 mg, 77%), δH (CDCl₃)/ppm 1.49 (9H, s), 3.20 (4H, br s), 3.56 (4H, br s), 6.84 (1H, d, J = 3.8 Hz), 7.30 (1 H, d, J = 8.8 Hz), 7.40 (1H, t, J = 7.9 Hz), 7.49-7.56 (2H, m), 7.65 (1H, d, J = 8.8 Hz), 7.70-7.76 (1H, m), 7.84 (1H, t, J = 1.9 Hz). MS: m/z (M-H)⁻ 508/5 10.

### Description 8

### 5-Methoxy-4-nitro-1-(toluene-4-sulfonyl)-1H-indole (D8)

To a solution of 5-methoxy-l-(toluene-4-sulfonyl)-1*H*-indole (for synthesis see: Zheng *et al.* Heterocycles 1994, 37 (3), 1761-1772) (2.0 g, 6.6 mmol) in DCM (20 ml) was added silica gel impregnated with nitric acid (see: Thummel *et al.* J. Org. Chem. 1993, **58** (7), 1668) (4.0 g) and the suspension stirred for 5 hours. The silica gel was then filtered off and washed with DCM (20 ml). The filtrate was concentrated *in vacuo* and purified by column chromatography over silica gel, eluting with DCM to afford the title compound (D8) (1.72 g, 75%), δH (CDCl₃)/ppm 2.36 (3H, s), 3.97 (3H, s), 6.92 (1H, d, J = 3.8 Hz), 7.07 (1H, d, J = 9.2 Hz), 7.25 (2H, d, J = 8.0 Hz), 7.67 ( 1 H, d, J = 4.0 Hz), 7.73 (2H, d, J = 8.0 Hz), 8.11 (1H, d, J = 9.0 Hz). MS: m/z (MH⁺) 347.

### Description 9

### 5-Metboxy-1-(toluene-4-sulfonyl)-1H-indol-4-ylamine (D9)

5-Methoxy-4-nitro-1-(toluene-4-sulfonyl)-1*H*-indole (D8) (1.0 g, 2.9 mmol) was dissolved in ethanol (50 ml) followed by addition of 10% palladium on carbon (0.2 g,). The resulting mixture was stirred under an atmosphere of hydrogen for 15 hours followed by filtration (Celite) and *in vacuo* removal of solvent. This provided the title compound (D9) (0.7 g, 77%), δH (CDCl₃)/ppm 2.33 (3H, s), 3.85 (3H, s), 3.95 (2H, br s), 6.54 (1H, d, J = 3.8 Hz), 6.87 (1H, d, J = 9.0 Hz), 7.19 (2H, d, J = 8.2 Hz), 7.34 (1H, d, J = 8.8 Hz), 7.43 (1H, d, J = 4.0 Hz), 7.72 (2H, d, J = 8.0 Hz). MS: m/z (MH⁺) 317.

### Description 10

### 2-Chloro-3,5-difluorobenzenesulfonyl chloride (D10)

A solution of sodium nitrite (0.44 g, 3.2 mmol) in water (2 ml) was added over 0.25 hours to a stirred, ice-cooled solution of 2-chloro-3,5-difluoroaniline (0.52 g, 3.2 mmol) in concentrated hydrochloric acid (7 ml). After stirring at this temperature for 20 minutes, the mixture was added in portions to a stirred mixture of copper (I) chloride (90 mg, 0.1 mmol) in a solution of glacial acetic acid saturated with sulfur dioxide gas. After the addition was complete, the mixture was extracted with DCM (2 x 30 ml) and the combined organic extracts were dried (MgSO₄) and concentrated *in vacuo* to give the title compound (0.38 g, 48%) (D10) as a crude oil which was used (see Example 131) without further purification.

### Description 11

### 2,3,5-Trichlorobenzenesulfonyl chloride (D11)

The title compound (D11) was prepared from 2,3,5-trichloroaniline (for preparation see Recl. Trav. Chimi. Pays-Bas, 1931, **50,** 112) by the method described in Description 10. The crude material was used in the next stage (see Example 130).

### Description 12

### 4-[1-(3-Chlorobenzeuesulfonyl)-1H-indol-4-yl]-piperazine-1-carboxylic acid tert-butyl ester (D12)

A 1 M solution of potassium tert-butoxide (60 ml, 60 mmol) was added over 15 minutes to a stirred solution of 4-(IH-indol-4-yl)-piperazine-J-carboxylic acid-tert-butyl ester [see WO 9967237 (1999) for preparation] (17.2 g, 57.1 mmol) in tetrahydrofuran (400 ml) at 8 °C under argon. The resulting solution was allowed to warm to ambient temperature over 30 minutes and then recooled to 8°C. To this cooled solution was then added a solution of 3-chlorobenzene sulfonyl chloride (12.5 g, 59.2 mmol) in tetrahydrofuran (100 ml) over 30 minutes. The solution was stirred at ambient temperature for 18 h under argon. After concentrating the reaction mixture to one third of the volume, ethyl acetate (700 ml) was added and the solution was washed with water (700 ml) and brine (700 ml). The organic phase was dried (MgSO₄) and concentrated *in vacuo* to give a residue which was stirred with diethyl ether (150 ml) to afford the title compound (D12) as a cream solid (22.1 g, 46.4 mmol, 81%), 8H (CDCl₃)/ppm 1.48 (9H, s), 3.07 (4H, m), 3.61 (4H, m), 6.70-6.74 (2H, m), 7.23-7.27 (1H, m), 7.3 7 (1H, t, J = 8.0 Hz), 7.49-7.52 (2H, m), 7.64 (1H, d, J = 8.0 Hz), 7.79 (1H, dd, J = 1.6, 8.0 Hz), 7.85 (1H, d, J = 1.8 Hz).

### Description 13

### 4-[5-Chloro-1-(3-chlorobenzenesulfonyl)-1H-indol-4-yl]-piperazine-1-carboxylic acid tert-butyl ester (D13)

N-Chlorosuccinimide (7.1 g, 53.2 mmol) was added portionwise over 0.75 h to a stirred solution of 4-[1-(3-chlorobenzenesulfonyl)-1*H*-indol-4-yl]-piperazine-1-carboxylic acid *tert*-butyl ester (D12) (22.0 g, 46.2 mmol) in glacial acetic acid (500 ml) at 28 °C under argon. After stirring the solution at ambient temperature for 2 days, dichloromethane (1.2 1) was added and the solution washed successively with water (11), 10% sodium thiosulfate solution (1.2 1), 5 M sodium hydroxide solution (2 x 1 I) and brine (1 1). The organic phase was dried (MgSO₄) and concentrated in vacuo to a foam residue. The residue was purified by chromatography over silica gel eluting with dichloromethane/hexane (4:1) followed by a gradient of dichtoromethane/ethyl acetate to afford the title compound (D13) (17.9 g, 35.1 mmol, 76%), δH (CDCl₃)/ppm 1.50 (9H, s), 3:2 (4H, br, s), 3.6 (4H, br, s), 6.84 (1H, d, J = 3.8 Hz) 7.30 (1H, d, J =8.8 Hz), 7.40 (1H, t, J = 8.0 Hz); 7:50-7.54 (2H, m), 7.65 (1H, d, J = 8.8 Hz), 7.73 (1H, dd, J = 2.6, 8.0 Hz), 7.84 (1H, d, J = 3.8 Hz).

### Description 14

### 4-(5-Chloro-1H-indol-4-yl)-piperazine-1-carboxylic acid tert-butyl ester (D14)

To a stirred solution of 4-(1*H*-indol-4-yl)-piperazine-1-carboxylic acid *tert*-butyl ester (for synthesis see: WO 9967237 (1999)) (6.9 g, 23 mmol) in dry 1,4-dioxane (100 ml) and water (0.041 ml) at 10 °C was added *N*-chlorosuccinimide (3.7 g, 28 mmol). The mixture was stirred in a water bath for 1.5 h during which time the temperature rose to 18 °C. Saturated aqueous sodium thiosulfate (15 ml) and saturated aqueous sodium bicarbonate (15 ml) were added. The resultant mixture was stirred vigorously for 10 min and then partitioned between *tert*-butyl methyl ether and brine. The organic layer was collected, concentrated *in vacuo* and purified by column chromatography over silica gel, eluting with a dichloromethane/*tert*-butyl methyl ether gradient to afford the title compound (D14) (5.5 g, 71%) which was identical to the material described in D6 as judged by comparison of the proton NMR spectra.

### Description 15

### 4-(7-Chloro-1H-indol-4-yl)-piperazine-1-carboxylic acid tert-butyl ester and 4-(3-chloro-1H-indol-4-yl)-piperazine-1-carboxylic acid tert-butyl ester mixture (ca. 9:1) (D15)

From the chromatographic purification for D14 was isolated (eluting after D14) a mixture of the title compounds (D15) (1.2 g, 16%), δH (CDCl₃)/ppm 1.50 (9H, s), 3.05-3.20 (4H, m), 3.62-3.70 (4H, m), 6.49-6.70 (2H, m), 7.04-7.23 (2H, m), 8.18 (0.1 H, br s), 8.42 (0.9H, br s). MS: m/z (M-H)- 334.

### Description 16

### 4-[3-Chloro-1-(3-chlorobenzenesulfonyl)-1H-indol-4-yl]-piperaxine-1-carboxylic acid tert-butyl ester (D16)

The mixture obtained from D15 (0.18 g) was converted to the sulfonamide as described for E₇₅₋E78. Column chromatography over silica gel, eluting with a dichloromethane/*tert*-butyl methyl ether gradient afforded the title compound (D16) (0.031 g) as a colourless, slowly solidifying oil. δH (CDCl₃)/ppm 1.48 (9H, s), 2.99 (4H, br s), 3.50-3.75 (4H, m), 6.85 (1H, d, J = 7.8 Hz), 7.28 (1H, t, J = 8.1 Hz), 7.40 (1H, t, J = 8.0 Hz), 7.49-7.55 (2H, m), 7.70 (1H, d, J = 8.4 Hz), 7.75 (1H, d, J = 8.0 Hz), 7.86 (1H, t, J = 0.9 Hz).

### Description 17

### 4-[7-Chloro-1-(3-chlorobenzenesulfonyl)-1H-indol-4-yl]-piperazine-1-carboxylic acid tert-butyl ester (D17)

From the chromatographic purification for D16 was isolated (eluting after D16) the title compound (D17) (0.059 g) as a colourless foam. δH (CDCl₃)/ₚₚₘ 1.49 (9H, s), 3.03-3.09 (4H, m), 3.61-3.67 (4H, m), 6.68 (1H, d, J = 8.4 Hz), 6.77 (1H, d, J = 4.0 Hz), 7.14 (1H, d, J = 8.4 Hz), 7.43 (1H, t, J = 8.0 Hz), 7.53-7.56 (1H, m), 7.69-7.72 (1H, m), 7.80 (1H, t, J = 1.8 Hz), 7.84 (1H, d, J=3.6Hz).

### Description 18

### 4-(7-Bromo-1H-indol-4-yl)-piperazine-1-carboxylic acid tert-butyl ester (D18)

To a stirred solution of 4-(1*H*-indol-4-yl)-piperazine-1-carboxylic acid *tert*-butyl ester (for synthesis see: WO 9967237 (1999)) (2.6 g, 8.5 mmol) in dry 1,4-dioxane (35 ml) and water (0.015 ml) at 10°C was added N-bromosuccinimide (1.8 g, 10 mmol). The mixture was allowed to warm to 25 °C over 1 h with stirring. Saturated aqueous sodium thiosulfate (5 ml) and saturated aqueous sodium bicarbonate (5 ml) were added . The resultant mixture was stirred vigorously for 5 min and then partitioned between *tert-*butyl methyl ether and halfsaturated brine. The organic layer was collected, concentrated *in vacuo* and purified by column chromatography over silica gel, eluting with a petroleum ether (40-60)/dichloromethane/*tert*-butyl methyl ether gradient to afford the title compound (D18) (1.2 g, 37%) as an off-white foam. δH (CDCl₃)/ppm 1.50 (9H, s), 3.12-3.20 (4H, m), 3.63-3.69 (4H, m), 6.48 (1H, d, J = 8.2 Hz), 6.62 (1H, dd, J = 3.0, 2.5 Hz), 7.21-7.29 (2H, m), 8.36 (1H, br s). MS: m/z (M-H)⁻ 378.

### Description 19

### 4-[7-Bromo-1-(3-chlorobenzenesulfonyl)-1H-indol-4-yl]-piperazine-1-carboxylic acid tert-butyl ester (D19)

To a stirred suspension of sodium hydride (67 mg, 60% suspension in oil) in dry THF (8 ml) was added dropwise 4-(7-bromo-l/y-indol-4-yl)-piperazine-l-carboxylic acid *tert*-butyl ester (D18) (0.32 g, 0.84 mmol) followed by 3-chlorobenzenesulfonyl chloride (0.24 ml).The mixture was sonicated for 25 min followed by the addition of dry DMF (5 ml), sodium hydride (34 mg, 60% suspension in oil) and 3-chlorobenzenesulfonyl chloride (0.12 ml). After 5 h more sodium hydride (67 mg, 60% suspension in oil) and 3-chlorobenzenesulfonyl chloride (0.24 ml) were added. After an additional 15 h the mixture was partitioned between *tert*-butyl methyl ether and aqueous sodium bicarbonate. The organic layer was washed with water and brine, concentrated *in vacuo* and purified by column chromatography over silica gel, eluting with a petroleum ether (40-60)/dichloromethane/*tert-*butyl methyl ether gradient to afford the title compound (D19) (0.14 g) as a slightly purple film. δH (CDCl₃)/ppm 1.49 (9H, s), 3.02-3.09 (4H, m), 3.62-3.69 (4H, m), 6.63 (1H, d, J = 8.4 Hz), 6.77 (1H, d, J = 8.8 Hz), 7.36 (1H, d, J = 8.4 Hz), 7.42 (1H, t, J = 7.9 Hz), 7.56 (1H, dt, J = 7.2, 1.1 Hz), 7.69 (1H, dt, J = 6.7, 0.7 Hz), 7.79 (1H, t, J = 1.8 Hz), 7.83 (1H, d, J = 3.8 Hz).

### Description 20

### 4-[7-Cyano-1-(3-chlorobenzenesulfonyl)-1H-indol-4-yl]-piperazine-1-carboxylic acid tert-butyl ester (D20)

A mixture of 4-[7-bromo-1-(3-chlorobenzenesulfonyl)-1*H*-indol-4-yl]-piperazine-1-carboxylic acid *tert*-butyl ester (D19) (0.13 g, 0.23 mmol), copper(I) cyanide (42 mg) and dry DMF (3 ml) was heated at reflux for 4 h. The volatiles were then evaporated *in vacuo* and to the residue were added water and saturated aqueous ammonia. The resultant mixture was extracted with ethyl acetate and *tert*-butyl methyl ether (2:1). The organic layer was washed with brine, concentrated *in vacuo* and purified by column chromatography over silica gel, eluting with a mixture of petroleum ether (40-60), dichloromethane and *tert*-butyl methyl ether to afford the title compound (D20) (0.063 g) as a colourless solid. δH (CDCl₃)/ppm 1.49 (9H, s), 3.18-3.24 (4H, m), 3 :63-3.68 (4H, m), 6.74 (1H, d, J = 8.3 Hz), 6.77 (1H, d, J = 3.8 Hz), 7.49 (1H, t, J = 8.0 Hz), 7.56-7.61 (2H, m), 7.79 (1H, d, J = 3.8 Hz), 7.92 (1H, t, J = 1.8 Hz), 8.02 (1H, dd, J = 8.0, 1.1 Hz) . MS: m/z (M-CO₂-C₄H₈ +1-1)⁺ 401.

### Description 21

### Nonafluorobutane-1-sulfonic acid-[5-cbloro-1-(3-cblorobenzenesulfonyl)-1H-indole-4-yl) ester (D21)

1,5,6,7-Tetrahydro-4*H*-indol-4-one (7.6 g, 56 mmol) was added to a suspension of sodium hydride (60 % in mineral oil, 2.5 g, 62 mmol) in dry DMF (150 ml), with cooling on an ice bath. This suspension was stirred for 20 minutes before adding 3-chlorobenzenesulfonyl chloride (13 g, 62 mmol). The reaction mixture was stirred at ambient temperature under argon for 16 h. *Tert-*butyl methyl ether and diethyl ether (*ca* 1:1, 500 ml) was added to the reaction mixture, which was then washed twice with water. The aqueous layer was extracted with diethyl ether and the combined ether extracts washed with brine, dried over MgSO₄ and evaporated to give a beige solid: 1-(3-Chlorobenzenesulfonyl)-1,5,6,7-tetrahydro-4*H*-indol-4-one (16.9 g); δH (CDCl₃)/ppm 2.13 (2H, m), 2.45 (2H, m), 2.99 (2H, m), 6.66 (1H, d, J = 3.5 Hz), 7.24 (1H, d, J = 3.5 Hz), 7.53 (1H, m), 7.65 (1H, d, J = 8.0 Hz), 7.74 (1H, d, J = 7.9 Hz), 7.86 (1H, s).
This material (16.9 g, 55 mmol) was processed as described [Heterocycles 1985, 23(1), 169] by heating with copper (II) chloride (36.5 g, 273 mmol) in 50% aqueous acetic acid (700 ml) for 2 h. Additional copper(II) chloride (36.5 g) was added and the reaction mixture heated for a further 16 h. After cooling, the mixture was poured into water and DCM, the aqueous layer extracted three times with DCM and the combined DCM extracts washed with saturated aqueous sodium bicarbonate, brine, dried (MgSO₄) and evaporated to give a brown oil which was purified by column chromatography to give the desired 5,5-dichloro-1-(3-chlorobenzenesulfonyl)-1,5,6,7-tetrahydro-4*H*-indol-4-one. This material (10.4 g, 28 mmol) was dissolved in DMF (600 ml). Lithium chloride (1.22 g, 29 mmol) and lithium carbonate (2.13 g, 29 mmol) were added and the mixture heated at 150 °C for 1 h, then concentrated *in vacuo.* The residue was poured into saturated aqueous ammonium chloride (800 ml) and extracted three times with DCM. The combined DCM layers were washed with water, dried (MgSO₄) and evaporated. Purification by column chromatography yielded the desired product 5-chloro-1-(3-chlorobenzenesulfonyl)-1*H-*indol-4-ol. This material (4.2 g, 12 mmol) was dissolved in THF (60 ml) and nonafluorobutyl-1-sulfonyl fluoride (4.5 g, 15 mmol) was added. Sodium hydride (60% in mineral oil, 0.50 g, 13 mmol) was added portionwise and the reaction mixture stirred under argon for 64 h. Ether (150 ml) and water (150 ml) were added, layers separated and the aqueous layer extracted with ether. The combined ether extracts were washed with brine, dried (MgSO₄) and evaporated to give a brown oil. This was purified using column chromatography to give the title compound (D21) (2.78 g) as a white solid; δH (CDCl₃)/ppm 6.81 (1H, dd, J = 3.8, 0.6 Hz), 7.45 (2H, m), 7.57 (1H, ddd, J = 1.1, 2.0, 7.9 Hz), 7.65 (1H, d, J = 3.8 Hz), 7.76 (1H, ddd, J =1.1, 1.8, 7.9 Hz), 7.88 (1H, t,J=1.8Hz),7.97(1H,dd,J=0.8Hz,8.8Hz).

### Description 22

### 4-[1-(3-Chlorobenzenesulfonyl)-5-cyano-1H-indol-4-yl]-piperazine-1-carboxylic acid tert-butyl ester (D22)

To a stirred solution of 4-[1-(3-chlorobenzenesulfonyl)-1H-indol-4-yl)-piperazine-1-carboxylic acid *tert*-butyl ester (D12) (350 mg, 0.74 mmol) in acetonitrile (6 ml) at 0 °C was added dropwise a solution of chlorosulfonylisocyanate (65 µl, 0.74 mmol) in acetonitrile (1 ml). After stirring for 1 h at this temperature, a solution of triethylamine (0.11 ml, 0.74 mmol) in acetonitrile (1 ml) was added dropwise and the reaction mixture allowed to warm to room temperature overnight. The mixture was then concentrated *in vacuo* and dissolved in chloroform. The organic phase was washed with saturated aqueous sodium bicarbonate solution, dried and evaporated *in vacuo.* Purification by column chromatography provided the title compound as a white solid (85 mg); δH (CDCl₃)/ppm 1.49 (9H, s), 3.43 (4H, t, J = 5.1 Hz); 3.63 (4H, t, J = 5.1 Hz), 6.83 (1 H, d, J = 3.8Hz), 7.44 (1H, t, J = 7.8 Hz), 7.46 (1H, d, J = 8.6 Hz), 7.56 (1H, ddd, J = 8.1, 2.0, 1.0 Hz), 7.59 (1H, d, J = 4.0 Hz), 7.68 (1H, d, J = 8.6 Hz), 7.77 (1 H, ddd, J = 7.8, 1.8, 1.0 Hz), 7.85 (1H, t, J = 1.8 Hz); MS: m/z (M-CO₂-C₄H₈ +H)⁺ 401.

### Description 23

### 4-[1-(3-Chlorobenzenesulfonyl)-2-methyl-1H-indol-4-yl]-piperazine-1-carboxylic acid tert-butyl ester (D23)

A solution of 4-[1-(3'-chlorobenzenesulfonyl)-1*H*-indol-4-yl]-piperazine-1-carboxylic acid *tert-*butyl ester (D12) (500 mg, 1.10 mmol) in THF (10 ml) was cooled to-78 °C followed by dropwise addition of a solution of t-BuLi in hexanes (0.74 ml, 1.3 mmol). The resulting mixture was allowed to stir at this temperature for 40 min followed by dropwise addition of iodomethane (0.70 ml, 11 mmol) in THF (5 ml). The reaction mixture was then allowed to stir and warm to room temperature overnight. Water was carefully added and the mixture extracted with diethyl ether. The extracts were dried and concentrated *in vacuo* followed by purification by preparative HPLC (details see E2-E73) to afford the title compound as, a clear paste (20 mg); δH (CDCl₃)/ppm 1.49 (9H, s), 2.60 (3H, s), 3.14 (4H, t, J = 4.8 Hz), 3.67 (4H, t, J = 4.8 Hz), 6.74-6.77 (2H, m), 7.17 (1H, t, J = 8.2 Hz), 7.23 (1H, t, J = 8.0 Hz), 7.36 (1H, d, J = 8.3 Hz), 7.56 (1H, d, J = 8.0 Hz), 7.62-7.65 (2H, m); MS: m/z (M-CO₂-C₄H₈ +H)⁺ 390.

### Description 24

### 4-Amino-6-cbloro-1H-indole (D24)

4-Chloro-2,6-dinitrotoluene (3.7 g, 17 mmol) (preparation see Helv. Chim. Acta 1936,19, 438.) was heated, at 110 °C with *N,N* dimethylformamide dimethyl acetal (6.8 ml) in DMF (20 ml) in a flask equipped with a reflux condenser for 6 h. Volatiles were evaporated *in vacuo,* the residue dissolved in toluene and again volatiles evaporated *in vacuo* to yield a deep red solid (4.6 g). A portion of this material (3.0 g) was dissolved in acetic acid. Titanium trichloride solution (14.5-15.5% in 10% aqueous hydrochloric acid, 141 ml) was added with stirring. After 7 min the mixture was poured onto ice with stirring. Sodium hydroxide (40% aqueous, 120 ml) followed by concentrated aqueous ammonia (120 ml) was carefully added with cooling in an ice bath. Isopropanol and DCM (1 : 3) were added, the resultant dark blue slurry filtered and the remaining solid washed well with 10% isopropanol in DCM. Combined filtrate and washings were concentrated *in vacuo* to yield the title compound (D24) (1.6 g, 9.6 mmol) as a brown oil. δH (CD₃OD)/ppm 6.22 (1H, d, J = 1.7 Hz), 6.37 (1H, dd, J = 3.3, 0.9 Hz), 6.66 (1H, dd, J = 1.7, 0.9 Hz), 6.97 (1H, d, J = 3.3 Hz); MS: m/z (M-H)⁻ 165.

### Description 25

### 4-(6-Chloro-1H-indol-4-yl)piperazine-1-carboxylic acid tert-butyl ester (D25)

4-Amino-6-chloro-1H-indole (D24) (2.1 g) was converted to crude 6-chloro-4-(4-methylpiperazin-1-yl)-1H-indole using the procedure from D1. Without purification the black oil thus obtained (5.3 g) was converted to crude 6-chloro-4-piperazin-1-yi-Itl-indole following the procedure given for E1. Without purification the product from this reaction was dissolved in 1,4-dioxane (45 ml). Saturated aqueous sodium bicarbonate solution (23 ml) and di-tert-butyl dicarbonate (5.5 g) were added. After vigorously stirring for 3 h the mixture was partitioned between brine and tert-butyl methyl ether. The organic layer was collected, concentrated and treated with MeOH (30 ml) and 40% aqueous sodium hydroxide (10 ml) for 2 h. This mixture was partitioned between water (200 ml) and *tert*-butyl methyl ether. The organic layer was washed with water and brine, concentrated *in vacuo* and submitted to column chromatography on silica gel, eluting with a *tert-*butyl methyl ether in dichloromethane gradient to afford the title compound (D25) (1.4 g) as an off-white foam. MS: m/z (M-H)⁻ 334.

### Description 26

### 4-[6-Chloro-1-(3-chlorobenzenesulfonyl)-1H-indol-4-yl]-piperazine-1-carboxylic acid tert-butyl ester (D26)

4-(6-Chloro-1*H*-indol-4-yl)-piperazine-1-carboxylic acid *tert*-butyl ester (D25) was converted to the sulfonamide as described for E75-E78. Column chromatography over silica gel, eluting with a dichloromethane/*tert*-butyl methyl ether gradient afforded the title compound (D26) as a colourless oil. δH (CDCl₃)/ppm 1.48 (9H, s), 3.05-3.22 (4H, m), 3.57-3.63 (4H, m), 6.66 (1H, d, J = 3.4 Hz), 6.70 (1H, d, J = 1.6 Hz), 7.41 (1H, t, J = 8.0 Hz), 7.49 (1H, d, J = 3.8 Hz), 7.53 (1H, ddd, J = 8.0, 1.8, 0.9 Hz), 7.67 (1H, d, J = 0.7 Hz), 7.76 (1H, dt, J = 7.9, 1.8 Hz), 7.84 (1H, t, J = 1.8 Hz).

### Description 27

### 4-Chloro-2-trifluoromethoxybenzenesulfonyl chloride (D27)

The title compound (D27) (yellow oil, 0.74 g) was prepared from 4-chloro-2-trifluoromethoxyaniline (0.82 g) by the method described in Description 10: δH (CDCl₃)/ppm 7.46-7.54 (2H, m), 8.05 (1H, d, J = 8.5 Hz).

### Description 28

### 4-Bromo-2-cyanobenzenesulfonyl chloride (D28)

The title compound (D28)(peach coloured powder, 0.74 g) was prepared from 4-bromo-2-cyanoaniline (0.97 g) by the method described in Description 10: δH (CDCl₃)/ppm 8.00 (1H, dd, J=8.6,1.9Hz),8.08(1H,d,J=8.6Hz),8.11(1H,d,J=1.9Hz).

### Description 29

### 4-(5-Methoxy-1H-indol-4-yl)-piperazine-1-carboxylic acid tert-butyl ester and 4-(7-methoxy-1H-indol-4-yl)-piperazine-1-carboxylic acid tert-butyl ester mixture (ca. 1:1) (D29)

4-Amino-5-methoxy-1*H*-indole was prepared by heating at reflux a mixture of 5-methoxy-1-(toluene-4-sulfonyl)-1*H*-indol-4-ylamine (0.5 g, 1.6 mmol) and aqueous sodium hydroxide (5 ml, 2N) in methanol (10 ml). After 24h, the mixture was allowed to cool to room temperature and concentrated *in vacuo.* This material (1.0 g) was converted to the mixture of title compounds (D29) (0.16 g brown oil) using the procedure from D25. MS: m/z (M-H)⁻ 330.

### Description 30

### 4-[5-Methoxy-1-(3-chlorobenzenesulfonyl)-1H-indol-4-yl]-piperazine-1-carboxylic acid tert-butyl ester (D30)

4-(5-Methoxy-1H-indol-4-yl)-piperazine-1-carboxylic acid *tert*-butyl ester and 4-(7-methoxy-1*H* indol-4-yl)-piperazine-1-carboxylic acid *tert*-butyl ester mixture (*ca.* 1:1) (D29) was converted to the respective sulfonamides as described for E75-E78. Column chromatography over silica gel, eluting with a *tert*-butyl methyl ether in dichloromethane and petroleum ether (40-60) gradient afforded the title compound (D30) (0.066 g) as a slightly brown oil. MS: m/z (M-CO₂-C₄H₈ +H)⁺ 406.

### Description 31

### 4-[7-Metboxy-1-(3-chlorobenzenesulfonyl)-1H-indol-4-yl]-piperazine-1-carboxylic acid tert-butyl ester (D31)

From the chromatographic purification for D30 was isolated (eluting after D30) the title compound (D31) (0.031 g) as a slightly brown oil. MS: m/z (M-CO₂-C₄H₈ +H)⁺ 406.

### Description 32

### Nonafluorobutane-1-sulfonic acid-(1-tert-butyloxycarbonyl-1H-indole-4-yl) ester (D32)

1*H*-Indol-4-ol (5.0 g) was dissolved in THF (150 ml) and nonafluorobutyl-1-sulfonyl fluoride (13 g) was added. With cooling in an ice bath sodium hydride (60% in mineral oil, 1.5 g) was added portionwise. The mixture was allowed to warm to room temperature over 3 h. Water was added and the mixture was twice extracted with ether. The combined organic extracts were washed with brine, dried (MgSO₄) and evaporated to give a green oil. This was purified using column chromatography (ethyl acetate in hexane gradient) to give nonafluorobutane-1-sulfonic acid-(1*H-*indole-4-yl) ester (10g). This material (4.8 g) was dissolved in DCM (20 ml) and di-*tert*-butyl dicarbonate (4.2 g) followed portionwise by 4-(dimethylamino)pyridine (1.9 g) were added. After 1 h the mixture was washed with aqueous HCl (0.5 M) and brine, dried (MgSO₄) and concentrated to give the title compound (D32) (6.2 g) as a white solid; δH (CDCl₃)/ppm 1.68 (9H, s), 6.68 (1H, d, J = 3.8 Hz), 7.18 (1H, d, J = 8.0 Hz), 7.33 (1H, t, J = 8.2 Hz), 7.67 (1H, d, J = 3.8 Hz), 8.20 (1H, d, J = 8.3 Hz).

### Description 33

### 4-(1-tert-Butyloxycarbonyl-1H-indol-4-yl)[1,4]diazepan-1-carboxylic acid tert-butyl ester (D33)

The title compound (D33) (0.79 g yellow oil) was obtained from nonafluorobutane-1-sulfonic acid-(1-*tert*-butyloxycarbonyl-1*H*-indole-4-yl) ester (D32) as described for E160; δH (CDCl₃)/ppm 1.44 and 1.47 (9H, 2 s), 1.65 (9H, s), 1.93-2.11 (2H, m), 3.40-3.73 (8H, m), 6.62 (1H, d, J = 3.2 Hz), 6.70 (1H, d, J = 7.8 Hz), 7.17 (1H, t, J = 8.1 Hz), 7.51 (1H, d, J = 3.8 Hz), 7.75 (1H, d, J = 8.3 Hz).

### Description 34

### 4-(1H-Indol-4-yl)-[1,4]diazepan-1-carboxylic acid tert-butyl ester (D34)

4-(1-*tert*-Butyloxycarbonyl-1*H*-indol-4-yl)-[1,4]diazepan-1-carboxylic acid *tert*-butyl ester (D33) (0.75 g) was heated with methanol (10 ml) and sodium methoxide in methanol (0.5 M, 11 ml) at reflux for 4 h and allowed to cool to 25 °C over 10 h. The mixture was concentrated *in vacuo,* dissolved in DCM and washed with water and brine, dried (MgSO₄) and concentrated *in vacuo* to afford the title compound (D34) (0.56 g) as a grey gum; δH (CDCl₃)/ppm 1.44 and 1.47 (9H, 2 s), 1.97-2.15 (2H, m), 3.43-3.75 (8H, m), 6.52 (1H, dd, J = 7.6, 0.6 Hz), 6.59 (1H, br s), 6.95 (1H, d, J = 8.1 Hz), 7.05 (1H, d, J = 7.8 Hz), 7.08-7.14 (1H, m), 8.13 (1H, br s).

### Description 35

### 4-[1-(3-Chlorobenzenesulfonyl)-1H indol-4-yl]-[1,4]diazepan-1-carboxylic acid tert-butyl ester (D35)

4-(1*H*-Indol-4-yl)-[1,4]diazepan-1-carboxylic acid *tert*-butyl ester (D34) was converted to the sulfonamide as described for E75-E78. Column chromatography over silica gel, eluting with a dichloromethane/hexane gradient afforded the title compound (D35) (0.42 g) as a yellow oil. δH (CDCl₃)/ppm 1.39 and 1.45 (9H, 2 s), 1.92-2.08 (2H, m), 3.38-3.68 (8H, m), 6.68 (1H, d, J = 8.0 Hz), 6.75 (1H, d, J = 3.7 Hz), 7.18 (1H, t, J = 8.1 Hz), 7.37 (1H, t, J = 7.9 Hz), 7.45-7.54 (3H, m), 7.75 (1H, dt, J = 7.8, 1.5 Hz), 7.85 (1H, t, 1.9 Hz).

### Description 36

### 4-(1-Triisopropylsitanyl-1H-indol-4-yl)-piperazine-1-carboxylic acid tert-butyl ester (D36)

To a suspension of sodium hydride (60%, 73 mg, 1.83 mmol) in THF (5 ml) was slowly added a solution of 4-(1*H*-indol-4-yl)-piperazine-1-carboxylic acid-*tert*-butyl ester [see WO 9967237 (1999) for preparation] (0.5 g, 1.6 mmol) in THF (5 ml) at 0°C. After stirring for 20 min, triisopropylsilyl chloride (39 µL, 1.83 mmol) was added dropwise and the mixture allowed to stir at room temperature for 4 h. Diethyl ether (50 ml) was then added and the organic phase washed with water, dried (MgSO₄) and solvent removed *in vacuo.* Column chromatography (DCM) provided the title compound (D36) as a clear oil (0.73 g). δH (CDCl₃)/ppm 1.14 (18H, d, J = 7.5 Hz), 1.50 (9H, s), 1.69 (3H, m), 3.18 (4H, t, J = 5.1 Hz), 3.66 (4H, t, J = 5.0 Hz), 6.57-6.62 (2H, m), 7.06 (1H, t, J = 7.6 Hz), 7.19-7.23 (2H, m).

### Description 37

### 4-(3-Dimethylaminometbyl-1-triisopropylsilanyl-1H-indol-4-yl)-piperazine-1-carboxylic acid tert-butyl ester (D37)

A mixture of 4-(1-triisopylsilanyl-1*H*-indol-4-yl)-piperazine-1-carboxylic acid *tert*-butyl ester (D36) (0.70 g, 1.53 mmol) and Eschenmoser's salt (0.28 g, 1.53 mmol) in acetonitrile (20 ml) was stirred at ambient temperature for 24 h. After addition of DCM (50 ml), the mixture was washed with saturated aqueous NaHCO₃ solution, water, dried (MgSO₄) and solvent removed *in vacuo.* Purification by column chromatography (0-10% methanol/DCM) gave the desired compound (D37) as an orange paste (0.62 g). δH (CDCl₃)/ppm 1.13 (18H, d, J = 7.5 Hz), 1.50 (9H, s), 1.70 (3H, m), 2.31 (6H, s), 3.01 (4H, br s), 3.64 (4H, br s), 3.95 (2H, s), 6.78 (1H, d, J = 7.5 Hz), 7.04 (1H, t, J = 8.1 Hz), 7. 19 (1H, s), 7.25 (1H, d, J =7.9 Hz).

### Description 38

### 4-(3-Methyl-1-triisopropylsilanyl-1H-indol-4-yl)-piperazine-1-carboxylic acid tert-butyl ester (D38)

To a solution of 4-(3-dimethylaminomethyl-1-triisopropylsilanyl-1*H*-indol-4-yl)piperazine-1-carboxylic acid *tert*-butyl ester (D37) (0.50 g, 0.97 mmol) in ethanol (20 ml) was added 10% Pd/C (0.10 g) and the mixture left to stir under an atmosphere of hydrogen gas. After 4 days, the mixture was filtered through Celite, solvent removed *in vacuo* and the crude material purified on silica (DCM) to give the title compound (D38) as ayellow oil (0.23 g) (CDCI,)/ppm 1.10 (18H, d, J = 7.5 Hz), 1.49 (9H, s), 1.66 (3H, m), 2.53 (3H, s), 3.02 (4H, br s), 3.63 (4H, br s), 6.69(1H,d,J=7.5Hz),6.92(1H,s),7.01(IKJ=8.0Hz),7.19(1H,d,J7.9Hz).

### Description 39

### 4-(3-Methyl-1H-indol-4-yl)-piperazine-1-carboxylic acid tert-butyl ester (D39)

4-(3-Methyl-1-triisopropylsilanyl-1*H*-indol4-yl)-piperazine-1-carboxylic acid *tert*-butyl ester (D38) (0.20 g, 0.21 mmol) was combined with tetra-n-butylammonium fluoride (0. 16 g, 0.25 mmol) in THF (10 ml) and the resulting mixture stirred at room temperature for 8 h. Diethyl ether (50 ml) was added and the organic phase washed with saturated aqueous NaHCO₃ solution, dried (MgSO₄) and solvent removed *in vacuo.* Column chromatography (DCM) provided the title compound (D39) as a clear paste (90 mg). δH (CDCl₃)/ppm 1.50 (9H, s), 2.53 (3H, s), 3.03 (4H, br s), 3.63 (4H, br s), 6.68 (1H, m), 6.90 (1H, s), 7.07 (2H, m), 7.95 (1H, br s).

### Description 40

### 4-[1-(3-Chlorobenzenesulfonyl)-3-methyl-1H-indol-4-yl]-piperazine-1-carboxylic acid tert-butyl ester (D40)

To a suspension of potassium *tert*-butoxide (43 mg, 0.38 mmol) in THF (2 ml) was added dropwise a solution of 4-(3-methyl-1*H*-indol-4-yl)-piperazine-1-carboxylic acid *tert*-butyl ester (D39) (80 mg, 0.25 mmol) in THF (2 ml). After stirring for 20 min at room temperature, a THF (1 ml) solution of 3-chlorobenzenesulfonyl chloride (80 mg, 0.38 mmol) was added and the mixture stirred at room temperature for 24 h. Diethyl ether (20 ml) was added and the organic phase washed with water, dried (MgSO₄) and solvent removed *in vacuo.* Purification on silica (DCM) led to the desired compound (D40) as a clear paste (48 mg). δH (CDCl₃)/ppm 1.48 (9H, s), 2.47 (3H, s), 2.92 (4H, br s), 3.60 (4H, br s), 6.88 (1H, d, J = 7.5 Hz), 7.23 (2H, m), 7.36 (1H, t, J = 8.0 Hz), 7.48 (1H, d, J = 8.1 Hz), 7.72 (2H, m), 7.84 (1H, s).

### Example 1

### 1-(5-Chloro-3-methyl-benzo[b]thiophene-2-sulfonyl)-4-piperazin-1-yl-1H-indole Oxalate (E1)

To a solution of 4-(4-benzyl-piperazin-1-yl)-1-(5-chloro-3-methyl-benzo[b]thiophene-2-sulfonyl)-*1H-*indole (D2) (93 mg, 0.17 mmol) in dry 1,2-dichloroethane (5 ml) was added *N,N-*diisopropylethylamine (0.16 ml, 0.9 mmol) and 1-chloroethyl chloroformate (0.09 ml, 0.9 mmol). The solution was stirred at 80°C under argon for 50 minutes then concentrated *in vacuo.* The residue was redissolved in methanol (10 ml) and the solution was refluxed for 1.3 hours under argon. After concentrating the mixture *in vacuo,* the residue was redissolved in DCM (15 ml) and the solution was washed with 10% potassium carbonate solution (15 ml) then water (2 x 15 ml). The organic phase was dried (MgSO₄) and concentrated *in vacuo* to a residue which was purified by column chromatography on silica gel eluting with a methanol/DCM gradient to afford the free base (55 mg) of the title compound (E1). Treatment of a solution of the free base (55 mg) in DCM (1 ml) with a solution of oxalic acid (1.5 equivalents) in methanol/diethyl ether gave the corresponding oxalate salt (E1) (63mg, 69%), δH (CD₃OD)/ppm 2.64 (3H, s), 3.29-3.45 (8H, m), 6.85-6.89 (2H, m), 7.28 (1H, t, J = 8.0Hz), 7.48 (1H, dd, J = 2.0, 8.7Hz), 7.71-7.74 (2H, m), 7.83-7.92 (2H, m); MH⁺ 446/448.

### Reaction Scheme for Reference

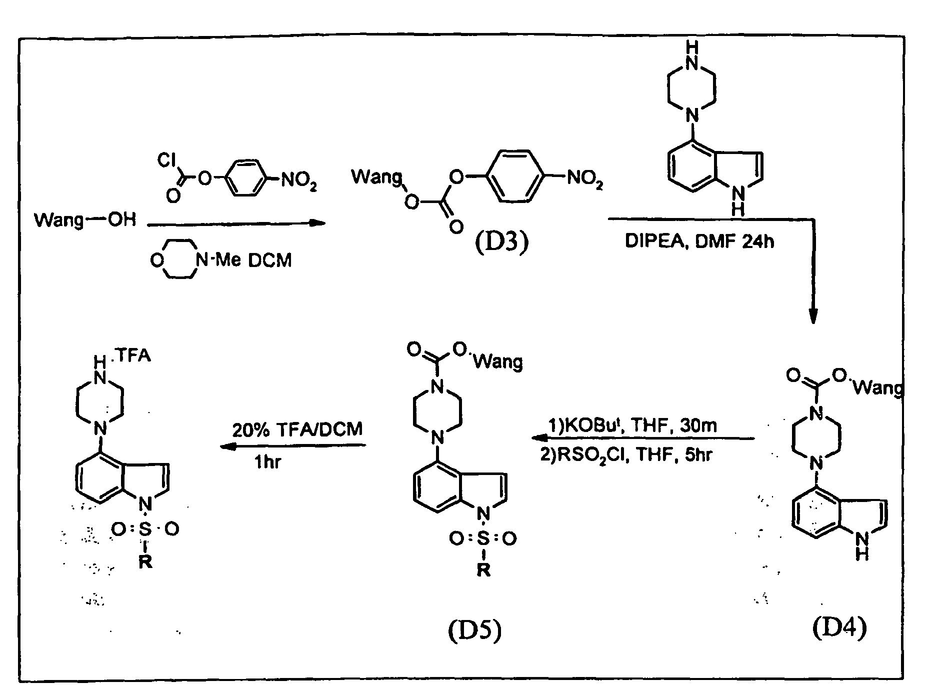

### General procedure for the preparation of 1-sulfonyl indoles by cleavage from resin

Each resin-bound 1-sulfonyl indole from Description 5 (assumed 0.14 mmol) was treated with DCM/trifluoroacetic acid (4:1, 1 ml) and agitated for 1 hour. The individual resin suspensions were then filtered into vials and washed with DCM (1 ml). The filtrates and washings for each resin were combined and evaporated *in vacuo* to give 1-sulfonyl indoles as trifluoroacetic acid salts. If required, further purification was performed on the Biotage Flex preparative HPLC (25 cm x 21.2 mm x 12 µl reverse phase eluted with acetonitrile/1% trifluoroacetic acid in water).

### Example 74

### 5-Chloro-1-(3-chloro-benzenesulfonyl)-4-piperazin-1-yl-1H-indole trifluoroacetic acid salt (E74)

A mixture of trifluoroacetic acid/DCM (5 ml, 10% v/v) was added to 4-[5-chloro-1-(3-chlorobenzenesulfonyl)-1*H*-indol-4-yl]-piperazine-1-carboxylic acid *tert*-butyl ester (D7) (40 mg, 78 mmol) and the resulting reaction mixture stirred for 4 hours before being concentrated *in vacuo* to provide the title compound (E74) (40 mg, 100%), δH (DMSO-d₆)/ppm 3.24 (4H, br s), 3.46 (4H, br s), 7.03 (1 H, d, J = 3.8 Hz), 7.40 (1 H, d, J = 8.8 Hz), 7.65 (1H, t, J = 8.0 Hz), 7.76-7.85 (2H, m), 7.97-8.00 (2H, m), 8.12 (1H, t, J = 1.8 Hz), 8.95 (2H, br s). MS: m/z (MH⁺) 409/411.

### Examples 75 - 78

### General procedure for the preparation of 1-arylsulfonyl-4-piperaxin-1-yl-5-chloro-1H-indoles

The title compounds E75-E78 (Table 2) were prepared from 4-(5-chloro-1*H* indol-4-yl)-piperazine-1-carboxylic acid *tert*-butyl ester (D6) and the corresponding arylsulfonyl chloride according to the procedure used in Description 7. This was followed by deprotection, as described in Example 74. The compounds were characterised by LC/MS (Table 2).

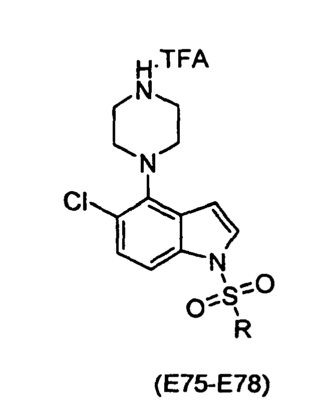

**Table 2**

| **Example** | **R** | **MH**^{**+**} |
|---|---|---|
| **5-Chloro-1-(3,5-dichlorobenzenesulfonyl)-4-piperazin-1-yl-1*H*-indole trifluoroacetic acid salt (E75)** | 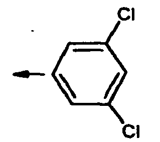 | 443/445 |
| **5-Chloro-1-(2,6-dichlorobenzenesulfonyl)-4-piperazin-1-yl-1*H*-indole trifluoroacetic acid salt (E76)** | 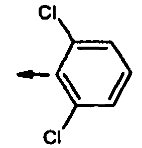 | 443/445 |
| **5-Chloro-1-(naphthalene-2-sulfonyl)-4-piperazin-1-yl-1*H*-indole trifluoroacetic acid salt (E77)** | 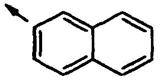 | 425/427 |
| **5-Chloro-1-(3,5-dichloro-2-methoxy benzenesulfonyl)-4-piperazin-1-yl-1*H*-indole trifluoroacetic acid salt (E78)** | 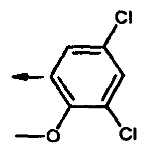 | 473/475 |

### General procedure for the preparation of 1-arylsulfonyl-4-piperazin-1-yl-1H-indotes via N-Boc deprotection

To a solution of 4-(1*H*-Indol-4-yl)-piperazine-1-carboxylic acid *tert*-butyl ester (0.1 g, 0.33 mmol) (for synthesis see: WO 9967237 (1999)) in THF (1 ml) was added a 1M solution of potassium tert-butoxide in THF (0.4 ml, 0.4 mmol) and the solution was stirred at room temperature for 10 minutes. A solution of the appropriate aryl sulfonyl chloride (0.66 mmol) in THF (1 ml) was then added and after 5 hours at room temperature, the reaction mixture was diluted with DCM (7 ml) and washed with saturated sodium hydrogen carbonate solution (2 x 7 ml). The organic phase was dried, concentrated and the resulting residue was purified by column chromatography over silica gel eluting with a gradient of ethyl acetate/hexane to give the corresponding intermediate N-Boc protected I-aryisulfonyl4-piperazin-1-yl-IH-indoles. These materials were deprotected by the method described in Example 74 to afford the title compounds.

### Example 132

### 5-Cbloro-1-(3-cblorobenzenesulfonyl)-4-piperazin-1-yl-1H-indole hydrochloride (E132)

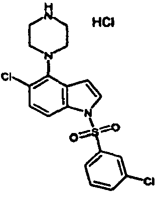

4-[5-Chloro-1-(3-chlorobenzenesulfonyl)-1H-indol-4-yl]-piperazine-1-carboxylic acid *tert*-butyl ester (D13) (17.2 g, 33.7 mmol) was dissolved in 1,4-dioxane (180 ml) and treated with an aqueous solution of hydrochloric acid (4 M, 180 ml). The cloudy mixture was warmed to 70 °C and stirred for two hours, then cooled and evaporated. The crystalline residue was recrystallised first from isopropanol then from methanol-water to afford the title compound (E132) as a white crystalline solid (10.5 g, 23.5 mmol, 70%): δH (CDCl₃)/ppm 3.4 (4H, br s), 3.6 (4H, br s), 4.8 (2H, br s), 6.9 (1H, d, J = 3.8 Hz), 7.23 (1H, d, J = 8.8 Hz), 7.52 (1H, t, J = 4.0 Hz), 7.65-7.76 (2H, m), 7.82 (1H, d, J = 8.8 Hz), 7.90 (1H, d, J = 8.4 Hz), 7.96 (1H, t, J = 1.8 Hz); m.p. (dec.) 240-241 °C (methanol-water) (crystal appearance changes at ca. 119 °C); Water (Karl Fischer) 2.7% w/w; Ionic chloride analysis: found 7.7% w/w (theoretical based on 2.7% w/w water 7.7%).

### Examples 135 -150

### General procedure for the preparation of 1-arylsulfonyl-5-c6loro.4-piperazin-1-yl-1H indole hydrochlorides

The title compounds E135-E150 (Table 5) were prepared from 4-(5-chloro-1*H*-indol-4-yl)-piperazine-1-carboxylic acid *tert*-butyl ester (D14) and the corresponding arylsulfonyl chlorides according to the procedure used in Description 7 followed by deprotection using 20 % TFA in DCM for 1 h. Evaporation *in vacuo,* treatment with excess HCl in diethyl ether (1 M) in the presence of methanol and evaporation *in vacuo* gave the title compounds which were characterised by LC/MS (Table 5).

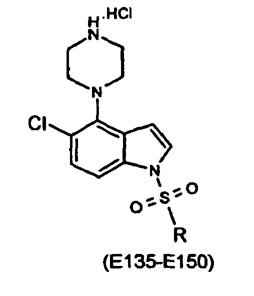

**Table 5**

| **Example** | **R** | **MH**⁺ |
|---|---|---|
| **1-(Benzenesulfonyl)-5-chloro-4-piperazin-1-yl-1*H*-indole hydrochloride (E135)** | 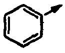 | 376 |
| **5-Chloro-1-(2,6-difluorobenzenesulfonyl)-4-piperazin-1-yl-1*H*-indole hydrochloride (E136)** | 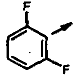 | 412 |
| **1-(2-Bromobenzenesulfonyl)-5-chloro-4-piperazin-1-yl-1*H*-indole hydrochloride (E137)** | 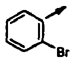 | 456 |
| **5-Chloro-1-(2-chlorobenzenesulfonyl)-4-piperazin-1-yl-1*H*-indole hydrochloride (E138)** | 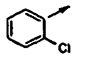 | 410 |
| **5-Chloro-1-(3-trifluoromethyl-benzenesulfonyl)-4-piperazin-1-yl-*H*-indole hydrochloride (E139)** | 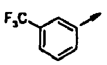 | 444 |
| **5-Chloro-1-(3-methylbenzenesulfonyl)-4-piperazin-1-yl-1*H*-indole hydrochloride (E140)** | 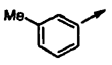 | 390 |
| **1-(3-Bromobenzenesulfonyl)-5-chloro-4-piperazin-1-yl-1*H*-indole hydrochloride (E141)** | 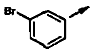 | 455 |
| **1-(4-Bromo-2-trifluoromethoxybenzenesulfonyl)5-chloro-4-piperazin-1-yl-1*H*-indole hydrochloride (E142)** | 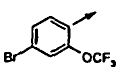 | 539 |
| **5-Chloro-1-(3-cyanobenzenesulfonyl)-4-piperazin-1-yl-1*H*-indole hydrochloride (E143)** | 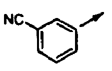 | 401 |
| **5 -Chloro-1-(1-naphthalenesulfonyl)-4-piperazin-1-yl-1*H*-**-**indole hydrochloride (E144)** | 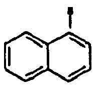 | 426 |
| **5-Chloro-1-(2,5-difluorobenzenesulfonyl)-4-piperazin-1-yl-1*H*-indole hydrochloride (E145)** | 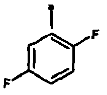 | 412 |
| **5-Chloro-1-(4-fluorobenzenesulfonyl)-4-piperaxin-1-yl-1*H* indole hydrochloride (E146)** | 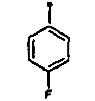 | 394 |
| **5-Chloro-1-(4-chlorobenzenesulfonyl)-4-piperazin-1-yl-1*H*-indole hydrochloride (E147)** | 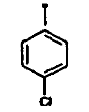 | 410 |
| **5-Chloro-1-(1,3,5-trimethyl-1*H*-pyrazole-4-sulfonyl)-4-piperazin-1-yl-1H-indole hydrochloride (E148)** | 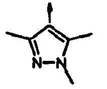 | 408 |
| **5-Chloro-1-(pyridine-2-sulfonyl)-4-piperazin-1-yl-1*H*-indole hydrochloride (E149)**^{**(a)**} | 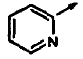 | 377 |
| **5-Chloro-1-(2,5-dimetbylbenzenesulfonyl)-4-piperazin-1-yl-1*H*-indole hydrochloride (E150)** | 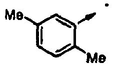 | 404 |

| | | |
|---|---|---|
| [a] From 2-pyridyl sulfonyl chloride [J. Org. Chem. 1989, 54 (2), 392.] | | |

### Example 151

### 3-Chloro-1-(3-chlorobenzenesulfonyl)-4-piperazin-1-yl-1H-indole hydrochloride (E151)

The title compound (E151) was obtained as a white solid following deprotection of D16 and hydrochloride salt formation as described for E135-E150. δH (CD₃OD)/ppm 3.24-3.32 (4H, m), 3.42-3.48 (4H, m), 7.04 (1H, d, J = 7.8 Hz), 7.41 (1H, t, J = 8.2 Hz), 7.56 (1H, t, J = 8.0 Hz), 7.69 (1H, dd, J = 7.1, 0.9 Hz), 7.82-7.88 (2H, m), 7.92 (1H, dd, J = 8.0, 1.0 Hz), 8.07 (1H, s). MS: m/z (M+H)⁺ 410.

### Example 152

### 7-Chloro-1-(3-chlorobenzenesulfonyl)-4-piperazin-1-yl-1H-indole hydrochloride (E152)

The title compound (E152) was obtained as a white solid following deprotection of D17 and hydrochloride salt formation as described for E135-E150. δH (CD₃OD)/ppm 3.28-3.36 (4H, m), 3.43-3.48 (4H, m), 6.87 (1H, d, J = 8.3 Hz), 6.96 (1H, d, J = 3.8 Hz), 7.21 (1H, d, J = 8.3 Hz), 7.56 (1H, t, J = 8.1 Hz), 7.70 (1H, ddd, J = 8.1, 2.0, 1.0 Hz), 7.76 (1H, ddd, J = 7.8, 1.8, 1.0 Hz), 7.83 (1 H, t, J = 1.8 Hz), 7.95 (1H, d, J = 3.8 Hz). MS: m/z (M+H)⁺ 410.

### Example 153

### 7-Chloro-1-(3,5-dichlorobenzenesulfonyl)-4-piperazin-1-yl-1H-indole hydrochloride (E153)

4-(7-Chloro-1*H*-indol-4-yl)-piperazine-1-carboxylic acid *tert*-butyl ester (D15, *ca.* 90% purity) (200 mg, 0.6 mmol) was dissolved in DMF (5 ml) and 3,5-dichlorobenzenesulfonyl chloride (147 mg, 0.6 mmol) added. Sodium hydride (60% in mineral oil, 72 mg, 1.8 mmol) was added and the reaction mixture stirred under argon for 16 h. The mixture was quenched with water, and extracted twice with ethyl acetate. The combined organic extracts were washed with brine, dried (MgS0₄) and evaporated. The crude residue was purified using column chromatography to give the N-tert-butyloxycarbonyl protected intermediate. This material was treated with 20 % trifluoroacetic acid in DCM for 1 h. Evaporation *in vacuo,* treatment with 1M HCI in diethyl ether and evaporation *in vacuo* gave the title compound (E153) as a colourless solid (85 mg), 8H (CD₃0D)/ppm 3.29 (8H, br s), 6.88 (1H, d, J = 8.4 Hz), 7.09 (1H, d, J = 3.9 Hz), 7.26 (1H, d, J = 8.4 Hz), 7.99 (3H, m), 8.09 (1H, m), 9.36 (2H, br s).

### Examples 154 - 155

### General procedure for the preparation of 1-arylsulfonyl-7-chloro-4-piperazin-1-yl-1H indole hydrochlorides

The title compounds E154-E155 (Table 6) were prepared from 4-(7-chloro-1*H*-indol-4-yl)-piperazine-1-carboxylic acid *tert*-butyl ester (D14) and the corresponding arylsulfonyl chlorides and converted to the hydrochloric acid salts according to the procedure used for E153 to give the title compounds (E154-E155) which were characterised by LC/MS (Table 5). E155 required purification by preparative HPLC (details see E2-E73) followed by treatment with hydrogen chloride (1 M solution in diethyl ether, 2 ml).

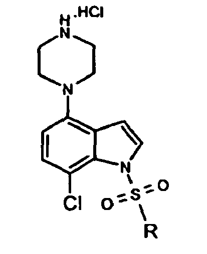

**Table 6**

| **Example** | **R** | **MH**^{**+**} |
|---|---|---|
| **1-Benzenesulfonyl-7-cbloro4-piperazin-1-yl-1*H*-indole hydrochloride (E154)** | 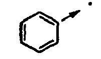 | 376 |
| **1-(4-BromG-2-trifluorometboxybenzenesulfonyl)-7-cbloro-4-piperazin-1-yl-1*H* indole hydrochloride (E155)** | 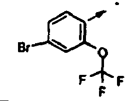 | 539 |

### Example 156

### 7-Bromo-1-(3-chlorobenzenesulfonyl)-4-piperazin-1-yl-1H-indole hydrochloride (E156)

4-(7-Bromo-1*H*-indol-4-yl)-piperazine-1-carboxylic acid *tert*-butyl ester (D18) (0.063 g) was allowed to react with hydrochloric acid (4 M in 1,4-dioxane, 2 ml) for 4 h. Volatiles were evaporated and the residue purified by preparative HPLC (details see E2-E73) followed by treatment with hydrogen chloride (1 M solution in diethyl ether, 1 ml) in the presence of methanol (2 ml) and evaporation to yield the title compound (E156) (0.019 g) as a colourless solid. δH (CD₃0D)/ppm 3.30-3.37 (4H, m), 3.43-3.50 (4H, m), 6.83 (1H, d, J = 8.3 Hz), 6.98 (1H, d, J = 3.9 Hz), 7.45 (1H, d, J = 8.3 Hz), 7.57 (1H, t, J = 8.0 Hz), 7.68-7.73 (1H, m), 7.73-7.80 (1H, m), 7.83 (1H, t, J = 1.9 Hz), 7.97 (1H, d, J = 3.9 Hz). MS: m/z (M+H)⁺ 454.

### Example 157

### 7-Cyano-1-(3-chlorobenzenesulfonyl)-4-piperazin-1-yl-1H-indole hydrochloride (E157)

The title compound (E157) was obtained as a white solid following deprotection of 4-[7-cyano-1-(3-chlorobenzenesulfonyl)-1*H*-indol-4-yl]-piperazine-1-carboxylic acid *tert*-butyl ester (D20) and hydrochloride salt formation as described for E156. δH (CD₃OD)/ppm 3.45-3.49 (4H, m), 3.49-3.54 (4H, m), 6.97 (1H, d, J = 8.3 Hz), 7.02 (1H, d, J = 3.8 Hz), 7.58 (1H, t, J = 8.1 Hz), 7.67 (1H, d, J = 8.3 Hz), 7.72 (1H, ddd, J = 8.1, 2.0, 1.0 Hz), 7.93 (1 H, ddd, J = 8.1, 2.0, 1.0 Hz), 7.96 (1H, d, J = 3.8 Hz), 8.05 (1H, t, J = 1.8 Hz). MS: m/z (M+H)⁺ 401/403.

### Example 158

### 1-(3-Chlorobenzenesulfonyl)-3,5-dichloro-4-piperazin-1-yl-1H-indole hydrochloride (E158)

To a stirred solution of 4-(1*H*-indol-4-yl)-piperazine-1-carboxylic acid *tert*-butyl ester (for synthesis see: WO 9967237 (1999)) (0.50 g, 1.7'mmol) in dry 1,4-dioxane (10 ml) and water (0.003 ml) at 10 °C was added N chlorosuccinimide (0.49 g). The mixture was allowed to warm to 25 °C with stirring over 4 h. Saturated aqueous sodium thiosulfate (5 ml) and saturated aqueous sodium bicarbonate (5 ml) were added. The resultant mixture was stirred vigorously for 5 min and then partitioned between *tert*-butyl methyl ether and water. The organic layer was washed with brine, concentrated *in vacuo* and purified by column chromatography over silica gel, eluting with a mixture of dichloromethane and *tert*-butyl methyl ether to afford a mixture of compounds (0.47 g) as judged by proton NMR spectroscopy. This was dissolved in dry THF (10 ml) and potassium *tert-*butoxide (1 M solution in THF, 1.5 ml) and 3-chlorobenzenesulfonyl chloride (0.22 ml) were added with stirring. After 2 h the resultant solution was partitioned between *tert*-butyl methyl ether and water containing brine and sodium bicarbonate. The organic layer was concentrated *in vacuo* and purified by column chromatography over silica gel, eluting with a mixture of petroleum ether (40-60), dichloromethane and *tert*-butyl methyl ether to afford a mixture of compounds (0.28 g) as a brown oil containing some solid. This was treated with trifluoroacetic acid for 30 min, the volatiles were then evaporated and the residue purified by preparative HPLC (details see E2-E73) followed by treatment with hydrogen chloride (1 M solution in diethyl ether, 2 ml) and evaporation to yield the title compound (E158) (0.080 g) as a colourless solid. δH (CD₃OD)/ppm 3.19-3.28 (2H, m), 3.28-3.48 (4H, m), 3.80-3.90 (2H, m), 7.45 (1H, d, J = 8.7 Hz), 7.52-7.62 (1H, m), 7.71 (1H, d, J = 8.3 Hz), 7.93-8.02 (3H, m), 8.05 (1H, s). MS: m/z (M+H)⁺ 444.

### Example 159

### 1-(3-Chlorobenzenesulfonyl)-5,7-dichloro-4-piperazin-1-yl-1H-indole hydrochloride (E159)

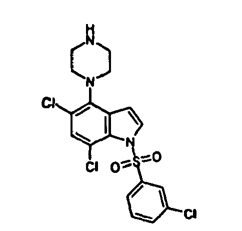

To a stirred solution of 4-[5-chloro-1-(3-chlorobenzenesulfonyl)-1*H*-indol-4-yl]-piperazine-1-carboxylic acid *tert*-butyl ester (D13) (0.63 g, 1.2 mmol) in dry 1,4-dioxane (10 ml) and water (0.004 ml) was added N-chlorosuccinimide (0.23 g). The mixture was stirred at 25°C for 1 h and at 80 °C for 15 h. Additional N-chlorosuccinimide (0.17 g) was added and heating continued for 8 h at 90°C. Saturated aqueous sodium thiosulfate (15 ml), saturated aqueous sodium bicarbonate (15 ml) and *tert*-butyl methyl ether were added. After stirring this mixture for 15 h the organic layer was collected, concentrated *in vacuo* and partially purified by preparative HPLC (details see E2-E73). The material thus obtained was allowed to react with di-*tert*-butyl dicarbonate in dioxane (0.5 M, 15 ml) and saturated aqueous sodium bicarbonate (8 ml) for 65 h. The mixture was partitioned between halfconcentrated brine and *tert*-butyl methyl ether. The organic layer was concentrated *in vacuo* and submitted to column chromatography over silica gel, eluting with a mixture of petroleum ether (40-60), dichloromethane and *tert*-butyl methyl ether to afford a mixture which was treated with trifluoroacetic acid (1.5 ml) in DCM (4 ml) for 30 min. Volatiles were evaporated and the residue purified by preparative HPLC (details see E2-E73) followed by treatment with hydrogen chloride (1 M solution in diethyl ether, 1 ml) in the presence of methanol (2 ml) and evaporation to yield the title compound (E159) (0.020 g) as a colourless solid. δH (DMSO-d₆)/ppm 3.26 (4H, br s), 3.42 (4H, br s), 7.15 (1H, d, J = 3.9 Hz), 7.45 (1H, s), 7.69 (1H, t, J = 8.0 Hz), 7.87-7.92 (2H, m), 8.02 (1H, t, J = 1. 8 Hz), 8.12 (1H, d, J = 1.9 Hz), 8.90 (2H, br s). MS: m/z (M+H)⁺ 444.

### Example 160

### 5-Chloro-1-(3-chlorobenzenesulfonyl)-4-[1,4]diazepan-1-yl-1H-indole hydrochloride (E160)

Palladium acetate (30 mg, 0.13 mmol), (±)-2,2'-bis(diphenylphosphino)-1,1'-binaphthyl (120 mg, 0.19 mmol), and cesium carbonate (590 mg, 1.8 mmol) in dioxane (20 ml) were sonicated at 35 °C for 30 minutes. The resulting deep red suspension was mixed with 1-*tert*-butyloxycarbonyl-[1, 4]diazepane (360 mg, 1.8 mmol) and nonafluorobutane-1-sulfonic acid-[5-chloro-1-(3-chlorobenzenesulfonyl)-1*H*-indole-4-yl] ester (D21) (700 mg, 1.1 mmol) in dioxane (5 ml) and the mixture heated to 95°C for 8 h. After cooling, the reaction mixture was concentrated and submitted to column chromatography to give the *N-tert*-butyloxycarbonyl protected intermediate (180 mg). This material was treated with 20 % trifluoroacetic acid in DCM for 30 min. Evaporation *in vacuo,* purification by preparative HPLC (details see E2-E73), treatment with 1M HCI in diethyl ether and evaporation *in vacuo* gave the title compound (E160) as a colourless solid (8 mg); MS: m/z (M+H)⁺ 424.

### Example 161

### 5-Chloro-1-(3-cblorobenzenesulfonyl)-4-((R)-3-metbylpiperazin-1-yl)-1H-indole (E161)

Palladium acetate (30 mg, 0.13 mmol), (±)-2,2'-bis(diphenylphosphino)-1,1'-binaphthyl (120 mg, 0.19 mmol), and cesium carbonate (590 mg, 1.8 mmol) in dioxane (20 ml) were sonicated at 35°C for 30 minutes. The resulting deep red suspension was mixed with (R)-2-methylpiperazine (185 mg, 1.8 mmol) and nonafluorobutane-1-sulfonic acid-[5-chloro-1-(3-chlorobenzenesulfonyl)-1*H*-indole-4-yl] ester (D21) (700 mg, 1.1 mmol) in dioxane (5 ml) and the mixture heated to 95°C for 8 h under argon. After cooling, the reaction mixture was concentrated and purified using column chromatography to give the title compound (E161) as a brown solid (27 mg); δH (CDCl₃)/ppm 1.17 (3H, m), 3.05-3.39 (7H, m), 6.87 (1H, br s), 7.30 (1H, d, J = 5.5 Hz), 7.40 (1H, m), 7.51 (2H, m), 7.66 (1H, d, J = 5.5 Hz), 7.74 (1H, d, J = 5.0 Hz), 7.84 (1H, m).; NH not visible.

### Example 162

### 5-Chloro-1-(3-chlorobenzenesulfonyl)-4-((S)-3-methylpiperazin-1-yl)-1H-indole (E162)

The title compound was obtained in the same way as for E161 using (*S*)-2-methylpiperazine instead of the (*R*)- isomer to give the title compound (E162) (69 mg) as a brown solid; δH (CDCl₃)/ppm 1.12 (3H, m), 2.98-3.32 (7H, m), 6.86 (1H, d, J=3.5 Hz), 7.30 (1H, m), 7.40 (1H, m), 7.50 (2H, m), 7.64 (1H, m), 7.71-7.76.(1H, m), 7.84 (1H, m); NH not visible.

### Example 164

### 1-(3-Chlorobenzenesulfonyl-5-cyano-4-piperazin-1-yl-1H-indole hydrochloride (E164)

The title compound (E164) was obtained as a colourless solid (60 mg) following deprotection of 4-[1-(3-chlorobenzenesulfonyl)-5-cyano-1*H*-indol-4-yl]-piperazine-1-carboxylic acid *tert-butyl* ester (D22) and hydrochloride salt formation as described for E135-E150. δH (CD₃OD)/ppm 3.42 (4H, t, J = 5.1 Hz), 3.69 (4H, t, J = 5.0 Hz), 7.04 (1H, dd, J = 3.9, 0.8 Hz), 7.50-7.60 (2H, m), 7.69 (1H, ddd, J = 8.1, 2.0, 1.0 Hz), 7.79 (1H, d, J = 4.2 Hz), 7.95-8.00 (2H, m), 8.01 (1H, t, J = 2.0 Hz); MS: m/z (M+H⁺) 401.

### Example 166

### 6-Chloro-1-(3-eblorobenzenesulfonyl)-4-piperazin-1-yl-1H-indole hydrochloride (E166)

The title compound (E166) was obtained as a white powder (0.18 g) following deprotection of 4-[6-chloro-1-(3-chlorobenzenesulfonyl)-1*H*-indol-4-yl]-piperazine-1-carboxylic acid *tert*-butyl ester (D26) (0.32 g) and hydrochloride salt formation as described for E135-E150. δH (CD₃OD)/ppm 3.35-3.38 (4H, m), 3.42-3.46 (4H, m), 6.88 (2H, s), 7.56 (1H, t, J = 8.0 Hz), 7.67-7.74 (3H, m), 7.90 (1H, d, J = 7.9 Hz), 7.96 (1H, s); MS: m/z (M+H⁺) 410.

### Example 169

### 5-Chloro-1-(4-chloro-2-trifluoromethoxybenzenesulfonyl)4-piperazin-1-yl-1H-indole hydrochloride (E169)

The title compound (E169) was obtained from 4-chloro-2-trifluoromethoxybenzenesulfonyl chloride (D27) and 4-(5-chloro-1H-indol-4-yl)-piperazine-1-carboxylic acid *tert*-butyl ester (D14) followed by deprotection and hydrochloride salt formation as described for E135-E150. MS: m/z (M+H⁺) 494.

### Example 170

### 1-(4-Bromo-2-cyanobenzenesulfonyl)-5-chloro-4-piperazin-1-yl-1H-indole hydrochloride (E170)

The title compound (E170) was obtained as a white solid from 4-bromo-2-cyanobenzenesulfonyl chloride (D28) and 4-(5-chloro-1H indol-4-yl)-piperazine-1-carboxylic acid *tert-*butyl ester (D14) followed by deprotection and hydrochloride salt formation as described for E135-E150. MS: m/z (M+H⁺) 479.

### Example 175

### 7-Chloro-1-(3-chlorobenzenesulfonyl)4-11,41diazepan-1-yl-IH-indole hydrochloride (E175)

To a stirred solution of -4-[1-(3-chlorobenzenesulfonyl)-*H*-indol-4-yl]-[1,4]diazepan-1 carboxylic acid *tert-*butyl ester (D35) (0.29 g) in dry 1,4-dioxane (20 ml) and water (2 pl) was added N-chlorosuccinimide (0.087 g). The mixture was stirred at 60 °C for 16 h. Additional N-chlorosuccinimide (0.045 g) was added and heating continued for 5 h. Saturated aqueous sodium thiosulfate (5 ml) and saturated aqueous sodium bicarbonate (5 ml) were added. The resultant mixture was stirred vigorously for 10 min and then extracted with *tert*-butyl methyl ether. The organic layer was collected, concentrated *in vacuo* and purified by column chromatography over silica gel, eluting with an ethyl acetate/ petroleum ether (40-60) gradient to give, eluting after 4-[5-chloro-1-(3-chlorobenzenesulfonyl)-1*H*-indol-4-yl]-[1,4]diazepan-1-carboxylic acid *tert*-butyl ester, a mixture of compounds containing 4-[7-chloro-1-(3-chlorobenzenesulfonyl)-1*H*-indol-4-yl]-[1,4]diazepan-1-carboxylic acid *tert*-butyl ester. This mixture was treated with TFA (2 ml) in DCM (2 ml) for 30 min followed by removal of volatiles *in vacuo.* Purification by preparative HPLC (details see E2-E73) followed by treatment with excess HCl in diethyl ether (1 M) in the presence of methanol and evaporation *in vacuo* yielded the title compound (E175) as an off-white solid (36 mg); δH (CD₃OD)/ppm 2.00-2.06 (2H, m), 3.21-3.30 (6H, m), 3.41-3.44 (2H, m), 6.63 (1H, d, J = 8.5 Hz), 6.71 (1H, d, J = 3.9 Hz), 6.94 (1H, d, J = 8.4 Hz), 7.34 (1 H, t, J = 8.0 Hz), 7.48 (1H, d, J = 8.2 Hz), 7.54 (1H, d, J = 8.0 Hz), 7.59 (1H, s), 7.70 (1H, d, J = 3.9 Hz); MS: m/z (M+H)⁺ 424.

### Pharmacological data

Compounds can be tested following the procedures outlined in WO 98/27081. All examples were found to have a pKi in the range 7.0 - 9.7 at human cloned 5-HT₆ receptors.

Furthermore, Examples E76-78, E132, E135, E137, E139-140, E149, E151-154, E156-159, E166, E170 and E175 were found to have a pKi in the range 8.8-9.7.

## Claims

1. A compound of formula (I) or a pharmaceutically acceptable salt thereof: 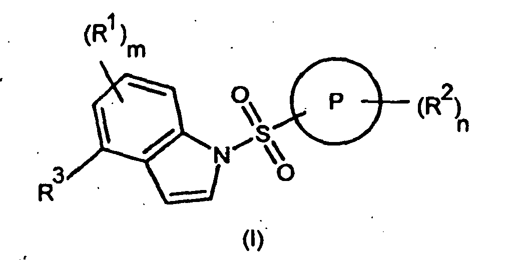 wherein:
P is phenyl, naphthyl or heteroaryl;
R¹ is chlorine, bromine or cyano;
R² is halogen, C₁₋₆alkyl. C₃₋₆cycloalkyl, C₁₋₆alkoxy, C₁₋₆alkylthio, C₁₋₆alkylsulphinyl, C₁₋₆alkylsulphonoyl, C₁₋₆alkanoyl, CN, CF₃, OCH₂CF₃, OCF₃, hydroxy, hydroxyC₁₋₆alkyl, hydroxyC₁₋₆alkoxy, C₁₋₆alkoxycarbonyl, C₁₋₆alkoxyC₁₋₆alkoxy, nitro, amino, N(C₁₋₆ alkyl)₂, NHC₁₋₆ alkyl, C₁₋₆alkylamino or diC₁₋₆alkylamino; or
R₂ is 4 group C(O)OR⁴, CONR⁵R⁶ or NR⁵COR⁶where R⁴ is hydrogen or C₁₋₆alkyl, and R⁵ and R⁶ are independently hydrogen, C₁₋₆alkyl or R⁵ and R⁶ combine together to form a 5- to 7- membered azacyclic ring optionally containing an additional heteroatom selected from nitrogen, sulphur or oxygen; or
R² is phenyl, naphthyl or heteroaryl optionally substituted by halogen, C₁-₆alkyJ, C₁₋₆alkoxy, C₁-₆alkanoyl, CN, CF₃, OCF₃, phenyloxy, benzyloxy or C₃₋₆cydloalkyloxy;
R³ is a 5- to 7-membered heterocyclic ring or a bicyclic heterocyclic ring containing 1 to 3 heteroatoms selected from nitrogen, sulphur or oxygen, said rings being optionally substituted by one or more C₁₋₆alkyl groups;
m is 1 or 2; or
n is 0-5.

2. A compound according to claim 1 in which R³ is an unsubstituted piperazine ring.

3. A compound according to claim 1 or 2 in which m is 1 and R¹ is chloro at the 5 position of the indole ring.

4. A compound according to any one of claims 1 to 3 in which m is 2 and R¹ is 5,7-dichloro.

5. A compound according to any one of claims 1 to 4 in which P is phenyl.

6. A compound according to any one of claims 1 to 5 in which n is 1 and R² is chlorine.

7. A compound according to claim 1 which is
5-Chloro-1-(3-chloro-benzenesulfonyl)-4-piperazin-1-yl-1*H*-indole;
5-Chloro-1-(3,5-dichlorobenzenesulfonyl)-4-piperazin-1-yl-1*H*-indole;
5-Chloro-1-(2,6-dichlorobenzenesulfonyl)-4-piperazin-1-yl-1*H*-indole;
5-Chloro-1-(naphthalene-2-sulfonyl)-4-piperazin-1-yl-1*H*-indole;
5-Chloro-1-(3,5-dichloro-2-methoxy benzenesulfonyl)-4-piperazin-1-yl-1*H* indole;
5-Chloro-1-(3-chlorobenzenesulfonyl)-4-piperazin-1-yl-1*H*-indole;
1-(Benzenesulfonyl)-5-chloro-4-piperazin-1-yl-1*H*-indole;
5-Chloro-1-(2,6-difluorobenzenesulfonyl)-4-piperazin-1-yl-1*H*-indole;
1-(2-Bromobenzenesulfonyl)-5-chloro-4-piperazin-1-yl-1*H*-indole;
5-Chloro-1-(2-chlorobenzenesulfonyl)-4-piperazin-1-yl-1*H*-indole;
5-Chloro-1-(3-trifluoromethyl-benzenesulfonyl)-4-piperazin-1-yl-1*H* indole;
5-Chloro-1-(3-methylbenzenesulfonyl)-4-piperazin-1-yl-1*H*-indole;
1-(3-Bromobenzenesulfonyl)-5-chloro-4-piperazin-1-yl-1*H* indole;
1-(4-Bromo-2-trifluoromethokybenzenesulfonyl)-5-chloro-4-piperazin-1-yl-1*H*-indole;
5-Chloro-1-(3-cyanobenzenesulfonyl)-4-piperazin-1-yl-1*H*-indole;
5-Chloro-1-(1-naphthalenesulfonyl)-4-piperazin-1-yl-1*H*-indole;
5-Chloro-l-(2.5-difluorobenzenesulfonyl)-4-piperazin-1-yl-IH-indole;
5-Chloro-l-(4-fluorobenzenesulfonyl)-4-piperazin-1-yl-1H indole;
5-Chloro-1-(4-chlorobenzenesulfony)-4-piperazin-1-yl-1*H*-indoLe:
5-Chloro-1 -(1.3.5-trimethyl-1*H*-pyrazole-4-sulfonyl)-4-piperazin-1 -yl-1*H*-indole:
5-Chloro-1-(pyridine-2-sulfonyl)-4-piperazin-1-yl-1*H*-indole;
5-Chloro-1 -(2,5-dimethylbenzenesulfonyl)-4-piperazin-I -yl-l *H*-indole;
3-Chloro-l-(3-chlorobenzenesulfonyl)-4-piperazin-1-yl-1 H-indole;
7 -Chloro-1-(3-chlorobenzenesulfonyl)-4-piperazin-1-yl-1 H-indole;
7-Chloro-1-(3,5-dichlorobenzenesutfonyl)-4-piperazin-1-yl-1H indole;
1-Benzenesulfonyl-7-chtoro-4-piperazin-1-yl-1H indole;
1-(4-Bromo-2-trifluoromethoxybenzenesulfonyl)-7-chloro-4-piperazin-1 -yl-l H-indole;
7-Bromo-1-(3-chlorobenzenesulfonyl)-4-piperazin-1-yl-1*H* indole;
7-Cyano-1-(3-chlorobenzenesulfonyl)-4-piperazin-1-yl-1*H*-indole;
1-(3-Chlorobenzenesulfonyl)-3,5-dichloro-4-piperazin-1-yl-1H-indole;
1-(3-Chlorobenzenesulfonyl)-5,7-dichloro-4-piperazin-1-yl-1H-indole;
5-Chloro-1-(3-chlorobenzenesulfonyl)-4-[1,4]diazepan-1-yl-1H indole;
5-Chloro-1-(3-chlorobenzenesulfonyl)-4-((R)-3-methylpiperazin-1-yl)-1H-indole;
5-Chloro-1-(3-chlorobenzenesulfonyl)-4-((S)-3-methylpiperazin-1-yl)-1 H-indole;
1-(3-Chlorobenzenesulfonyl)-5-cyano-4-piperazin-1-yl-1 H-indole;
6-Chloro-1-(3-chlorobenzenesulfonyl)-4-piperazin-1-yl-1 H-indole;
5-Chloro-1.-(4-chloro-2-trifluoroniethoxybenzenesulfonyl)-4-piperazin-1-yl-1*H*-indole;
1-(4-Bromo-2-cyanobenzenesulfonyl)-5-chloro-4-piperazin-1-yl-1H-indole;
7-Chloro-1-(3-chlorobenzenesulfonyl)-4-[1,4]diazepan-1-yl-1*H*-indole;
or a pharmaceutically acceptable salt thereof.

8. A pharmaceutical composition comprising a compound of formula (I) as defined in any one of claims 1 to 7 or a pharmaceutically acceptable salt thereof and a pharmaceutically acceptable carrier or excipient.

9. Use of a compound of formula (I) as defined in any one of claims 1 to 7 in the manufacture of a medicament for the treatment of depression, anxiety, cognitive memory disorders, Alzheimers disease, age related cognitive decline, mild cognitive impairment, ADHD and/or schizophrenia.

10. A process for the preparation of a compound of formula (I) as defined in claim 1 or a pharmaceutically acceptable salt thereof, which process comprises
(a) the coupling of a compound of formula (II): 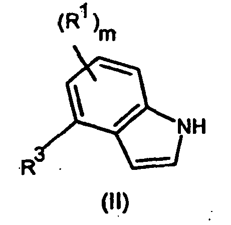 in which R¹, R³ and m are as defined in formula (I) or protected derivatives thereof with a-compound of formula (III): 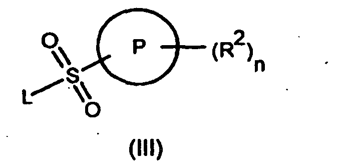 in which P, R² and n are as defined in formula (I) or a protected derivative thereof and L is a leaving group and optionally thereafter:
• removing any protecting groups,
• forming a pharmaceutically acceptable salt;
(b) preparing a compound of formula (I) wherein R³ represents an optionally substituted piperazinyl or 1,4-diazepanyl group linked to the indole moiety via a nitrogen atom which comprises reacting a compound of formula (IV) 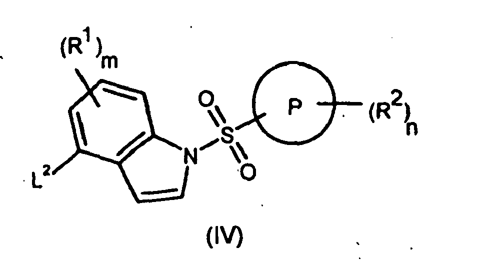 wherein R¹, R², P, m and n are as defined in formula (I) or a protected derivative thereof, and L² represents a suitable leaving group (eg. a halogen atom such as bromine or a trifluoromethylsulfonyloxy or nonafluorobutylsulfonyloxy group), with a compound of R^{3'-}H, wherein R^{3'} represents an optionally protected and/or substituted piperazinyl or 1,4-diazepanyt group and optionally thereafter:
• removing any protecting groups,
• forming a pharmaceutically acceptable salt;.or
(c) deprotecting a compound of formula (I) which is protected; or
(d) interconversion of a compound of formula (1) to other compounds of formula (I). ' ,

## Patentansprüche

1. Verbindung der Formel (I) oder ein pharmazeutisch verträgiches Salz davon: 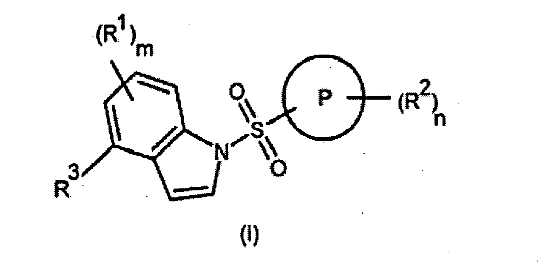 wobei:
P Phenyl, Naphthyl oder Heteroaryl ist;
R¹ Chlor, Brom oder Cyano ist;
R² Halogen, C₁₋₆-Alkyl, C₃₋₆-Cycloalkyl, C₁₋₆-Alkoxy, C₁₋₆-Alkylthio, C₁₋₆-Alkylsulfinyl, C₁₋₆-Alkylsulfonyl, C₁₋₆-Alkanoyl, CN, CF₃, OCH₂CF₃, OCF₃, Hydroxy, Hydroxy-C₁₋₆₋alkyl, Hydroxy-C₁₋₆-alkoxy, C₁₋₆-Alkoxycarbonyl, C₁₋₆-Alkoxy-C₁₋₆-alkoxy, Nitro, Amino, N(C₁₋₆-Akyl)₂, NHC₁₋₆-Alkyl, C₁₋₆-Alkylamino oder Di-C₁₋₆-Alkylamino ist; oder R² ein Rest C(O)OR⁴, CONR⁵R⁶ oder NR⁵COR⁶ ist, wobei R⁴ Wasserstoff oder C₁₋₆-Alkyl ist und R⁵ und R⁶ unabhängig voneinander Wasserstoff, C₁-₆-Alkyl sind oder R⁵ und R⁶ miteinander verbunden sind, um einen 5- bis 7-gliedrigen azacyclischen Ring zu bilden, welcher gegebenenfalls ein zusätzliches Heteroatom, ausgewählt aus Stickstoff, Schwefel oder Sauerstoff, enthält; oder
R² Phenyl, Naphthyl oder Heteroaryl ist, gegebenenfalls substituiert mit Halogen, C₁₋₆-Alkyl, C₁₋₆-Alkoxy, C₁₋₆-Alkanoyl, CN, CF₃, OCF₃, Phenyloxy, Benzyloxy oder C₃₋₆-Cycloalköxy;
R³ ein 5- bis 7-gliedriger heterocyclischer Ring oder ein bicyclischer heterocyclischer Ring ist, welcher 1 bis 3 Heteroatome enthält, ausgewählt aus Stickstoff, Schwefel oder Sauerstoff, wobei die Ringe gegebenenfalls mit einem oder mehreren C₁₋₆-Alkylresten substituiert sind;
m gleich 1 oder 2 ist; oder
n gleich 0 bis 5 ist.

2. Verbindung gemäß Anspruch 1, wobei R³ ein unsubstituierter Piperazinring ist.

3. Verbindung gemäß Anspruch 1 oder 2, wobei m gleich 1 ist und R¹ Chlor an der Position 5 des Indolrings ist.

4. Verbindung gemäß einem der Ansprüche 1 bis 3, wobei m gleich 2 ist und R¹ 5,7-Dichlor ist.

5. Verbindung gemäß einem der Ansprüche 1 bis 4, wobei P Phenyl ist.

6. Verbindung gemäß einem der Ansprüche 1 bis 5, wobei n gleich 1 ist und R² Chlor ist.

7. Verbindung gemäß Anspruch 1, nämlich:
5-Chlor-1-(3-chlorbenzolsulfonyl)-4-piperazin-1-yl-1*H*-indol;
5-Chlor-1-(3,5-dichlorbenzolsulfonyl)-4-piperazin-1-yl-1*H*-indol;
5-Chlor-1-(2,6-dichlorbenzolsulfonyl)-4-piperazin-1-yl-1*H*-indol;
5-Chlor-1-(naphthalin-2-sulfonyl)-4-piperazin-1-yl-1*H*-indol;
5-Chlor-1 -(3,5-dichlor-2-methoxybenzolsulfonyl)-4-piperazin-1-yl-1*H*-indol;
5-Chlor-1-(3-chlorbenzolsulfonyl)-4-piperazin-1-yl-1*H*-indol;
1-(Benzolsulfonyl)-5-chlor-4-piperazin-1-yl-1*H*-indol;
5-Chlor-1-(2,6-difluorbenzolsulfonyl)-4-piperazin-1-yl-1*H*-indol;
1-(2-Brombenzolsulfonyl)-5-chlor-4-piperazin-1-yl-1H-indol;
5-Chlor-1-(2-chlorbenzolsulfonyl)-4-piperazin-1-yl-1H-indol;
5-Chlor-1-(3-trifluormethylbenzolsulfonyl)-4-piperazin-1-yl-1H-indol;
5-Chlor-1-(3-methylbenzolsulfonyl)-4-piperazin-1-yl-1H-indol;
1-(3-Brombenzolsulfonyl)-5-chlor-4-piperazin-1-yl-1H-indol;
1-(4-Brom-2-trifluormethoxybenzolsulfonyl)-5-chlor-4-piperazin-1-yl-1H-indol;
5-Chlor-1-(3-cyanobenzolsulfonyl)-4-piperazin-1-yl-1*H*-indol;
5-Chlor-1-(1-naphthalinsulfonyl)-4-piperazin-1-yl-1*H*-indol;
5-Chlor-1-(2,5-difluorbenzolsulfonyl)-4-piperazin-1-yl-1*H*-indol;
5-Chlor-1-(4-fluorbenzolsulfonyl)-4-piperazin-1-yl-1*H*-indol;
5-Chlor-1-(4-chlorbenzolsulfonyl)-4-piperazin-1-yl-1*H*-indol;
5-Chlor-1-(1,3,5-trimethyl-1*H*-pyrazol-4-sulfonyl)-4-piperazin-1-yl-1*H*-indol;
5-Chlor-1-(pyridin-2-sulfonyl)-4-piperazin-1-yl-1*H*-indol;
5-Chlor-1-(2,5-dimethylbenzolsulfonyl)-4-piperazin-1-yl-1*H*-indol;
3-Chlor-1-(3-chlorbenzolsulfonyl)-4-piperazin-1-yl-1*H*-indol;
7-Chlor-1-(3-chlorbenzolsulfonyl)-4-piperazin-1-yl-1*H*-indol;
7-Chlor-1-(3,5-dichlorbenzolsulfonyl)-4-piperazin-1-yl-1H-indol;
1-Benzolsulfonyl-7-chlor-4-piperazin-1-yl-1H-indol;
1-(4-Brom-2-trifluormethoxybenzolsulfonyl)-7-chlor-4-piperazin-1-yl-1H-indol;
7-Brom- 1-(3-chlorbenzolsulfonyl)-4-piperazin- 1 -yl- 1*H*-indol;
7-Cyano=1-(3-chlorbenzolsulfonyl)-4-piperazin-1-yl-1H-indol; .
1-(3-Chlorbenzolsulfonyl)-3,5-dichlor-4-piperazin-l-yl-1H-indol;
1-(3-Chlorbenzolsulfonyl)-5,7-dichlor-4-piperazin-1-yl-IH-indol;
5-Chlor-1-(3-chlorbenzolsulfonyl)-4-[1,4]diazepan-1-yl-1H-indol;
5-Chlor-1-(3-chlorbenzolsulfonyl)-4-((R)-3-methylpiperazin-1-yl)-1H-indol;
5-Chlor-1-(3-chlorbenzolsulfonyl)-4-((S)-3-methylpiperazin-1-yl)-1H-indol;
1-(3-Chlorbenzolsulfonyl)-5-cyano-4-piperazin-1-yl-1*H*-indol;
6-Chlor-1-(3-chlorbenzolsulfonyl)-4-piperazin-1-yl-1*H*-indol;
5-Chlor-1-(4-chlor-2-trifluormethoxybenzolsulfonyl)-4-piperazin-1-yl-1*H*-indol;
1-(4-Brom-2-cyanobenzolsulfonyl)-5-chlor-4-piperazin-1-yl-1*H*-indol;
7-Chlor-1-(3-chlorbenzolsulfonyl)-4-[1,4]diazepan-1-yl-1*H*-indol;
oder ein pharmazeutisch verträgliches Salz davon.

8. Arzneimittel, umfassend eine Verbindung der Formel (I), wie in einem der Ansprüche 1 bis 7 definiert, oder ein pharmazeutisch verträgliches Salz davon und einen pharmazeutisch verträglichen Träger oder Exzipienten.

9. Verwendung einer Verbindung der Formel (I), wie in einem der Ansprüche 1 bis 7 definiert, zur Herstellung eines Medikaments zur Behandlung von Depression, Angst, kognitiven Gedächtnisstörungen, Alzheimer Krankheit, mit dem Alter verbundenem kognitiven Verfall, schwacher kognitiver Verschlechterung, ADHD und/oder Schizophrenie.

10. Verfahren zur Herstellung einer Verbindung der Formel (I), wie in Anspruch 1 definiert, oder eines pharmazeutisch verträglichen Salzes davon, wobei das Verfahren umfasst
(a) das Kuppeln einer Verbindung der Formel (II): 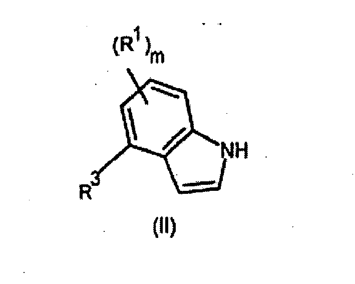 wobei R¹, R³ und m wie in Formel (I) definiert oder geschützte Derivate davon sind, mit einer Verbindung der Formel (III): 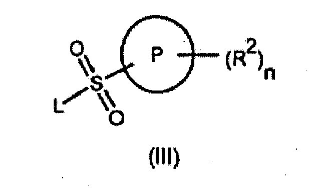 wobei P, R² und n wie in Formel (I) definiert oder ein geschütztes Derivat davon sind und L eine Abgangsgruppe ist, und gegebenenfalls anschließend:
• Entfernen jeglicher Schutzgruppen,
• Bilden eines pharmazeutisch verträglichen Salzes;
(b) Herstellen einer Verbindung der Formel (I), wobei R³ einen gegebenenfalls substituierten Piperazinyl- oder 1,4-Diazepanylrest darstellt, gebunden an die Indoleinheit über ein Stickstoffatom, welches das Umsetzen einer Verbindung der Formel (IV) 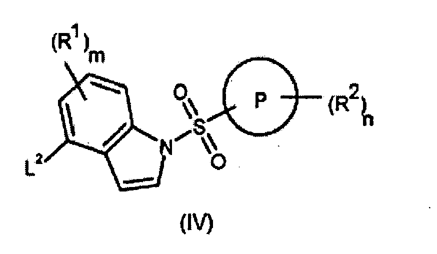 wobei R¹, R² P, m und n wie in Formel (I) definiert oder ein geschütztes Derivat davon sind und L² eine geeignete Abgangsgruppe (z.B. ein Halogenatom, wie Brom, oder einen Trifluormethylsulfonyloxy- oder Nonafluorbutylsulfonyloxyrest) darstellt, mit einer Verbindung R^{3'}-H umfasst, wobei R³' einen gegebenenfalls geschützten und/oder substituierten Piperazinyl- oder 1,4-Diazepanylrest darstellt, und gegebenenfalls anschließend:
• Entfernen jeglicher Schutzgruppen,
• Bilden eines pharmazeutisch verträglichen Salzes; oder
(c) Entschützen einer Verbindung der Formel (I), welche geschützt ist; oder
(d) Umwandlung einer Verbindung der Formel (I) in andere Verbindungen der Formel (I).

## Revendications

1. Composé de formule (I) ou un de ses sels pharmaceutiquement acceptables : 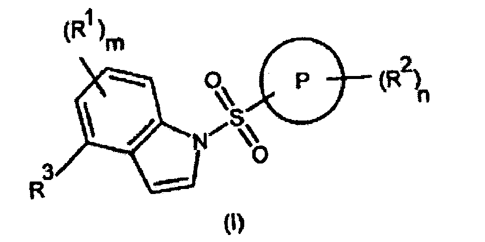 formule dans laquelle :
P représente un groupe phényle, naphtyle ou hétéroaryle ;
R¹ représente un atome de chlore ou de brome ou un groupe cyano ;
R² représente un atome d'halogène, un groupe alkyle en C₁ à C₆, cycloalkyle en C₃ à C₆, alkoxy en C₁ à C₆, alkylthio en C₁ à C₆, alkylsulfinyle en C₁ à C₆, alkylsulfonyle en C₁ à C₆, alcanoyle en C₁ à C₆, CN, CF₃, OCH₂CF₃, OCF₃, hydroxy, hydroxyalkyle en C₁ à C₆, hydroxyalkoxy en C₁ à C₆, (alkoxy en C₁ à C₆) carbonyle, (alkoxy en C₁ à C₆) (alkoxy en C₁ à C₆), nitro, amino, N (alkyle en C₁ à C₆)₂, NH (alkyle en C₁ à C₆), alkylamino en C₁ à C₆ ou di (alkyle en C₁ à C₆) amino ; ou bien
R² représente un groupe C(O)OR⁴, CONR⁵R⁶ ou NR⁵COR⁶, dans lequel R⁴ représente l'hydrogène ou un groupe alkyle en C₁ à C₆, et R⁵ et R⁶ représentent indépendamment l'hydrogène, un groupe alkyle en C₁ à C₆ ou bien R⁵ et R⁶ sont combinés l'un à l'autre pour former un noyau azacyclique penta- à heptagonal contenant facultativement un hétéroatome supplémentaire choisi entre l'azote, le soufre et l'oxygène ; ou bien
R² représente un groupe phényle, naphtyle ou hétéroaryle facultativement substitué avec un substituant halogéno, alkyle en C₁ à C₆, alkoxy en C₁ à C₆, alcanoyle en C₁ à C₆, CN, CF₃, OCF₃, phényloxy, benzyloxy ou cycloalkyloxy en C₃ à C₆ ;
R³ représente un noyau hétérocyclique penta- à heptagonal ou un noyau hétérocyclique bicyclique contenant 1 à 3 hétéroatomes choisis entre l'azote, le soufre et l'oxygène, lesdits noyaux étant facultativement substitués avec un ou plusieurs groupes alkyle en C₁ à C₆;
m est égal à 1 ou 2 ; ou
n a une valeur de 0 à 5.

2. Composé suivant la revendication 1, dans lequel R³ représente un noyau pipérazine non substitué.

3. Composé suivant la revendication 1 ou 2, dans lequel m est égal à 1 et R¹ représente un groupe chloro en position 5 du noyau indole.

4. Composé suivant l'une quelconque des revendications 1 à 3, dans lequel m est égal à 2 et R¹ représente un groupe 5,7-dichloro.

5. Composé suivant l'une quelconque des revendications 1 à 4, dans lequel P représente un groupe phényle.

6. Composé suivant l'une quelconque des revendications 1 à 5, dans lequel n est égal à 1 et R² représente le chlore.

7. Composé suivant la revendication 1, qui est le 5-chloro-1-(3-chloro-benzènesulfonyl)-4-pipérazine-1-yl-1H-indole ;
5-chloro-1-(3,5-dichlorobenzènesulfonyl)-4-pipérazine-1-yl-1H-indole ;
5-chloro-1-(2,6-dichlorobenzènesulfonyl)-4-pipérazine-1-yl-1H-indole ;
5-chloro-1-(naphtalène-2-sulfonyl)-4-pipérazine-1-yl-1H-indole ;
5-chloro-1-(3,5-dichloro-2-méthoxybenzènesulfonyl)-4-pipérazine-1-yl-1H-indole ;
5-chloro-1-(3-chlorobenzènesulfonyl)-4-pipérazine-1-yl-1*H-*indole ;
1-(benzènesulfonyl)-5-chloro-4-pipérazine-1-yl-1*H*-indole ;
5-chloro-1-(2,6-difluorobenzènesulfonyl)-4-pipérazine-1-yl-1H-indole ;
1-(2-bromobenzènesulfonyl)-5-chloro-4-pipérazine-1-yl-1H-indole ;
5-chloro-1-(2-chlorobenzènesulfonyl)-4-pipérazine-1-yl-1H-indole ;
5-chloro-1-(3-trifluorométhyl-benzènesulfonyl)-.4-pipérazine-1-yl-lh-indole ;
5-chloro-1-(3-méthylbenzènesulfonyl)-4-pipérazine-1-yl-1H-indole ;
1-(3-bromobenzènesulfonyl)-5-chloro-4-pipérazine-1-yl-1*H-*indole ;
1-(4-bromo-2-trifluorométhoxybenzènesulfonyl)-5-chloro-4-pipérazine-1-yl-1H-indole ;
5-chloro-1-(3-cyanobenzènesulfonyl)-4-pipérazine-1-yl-1*H-*indole ;
5-chloro-1-(1-naphtalènesulfonyl)-4-pipérazine-1-yl-1H-indole ;
5-chloro-1-(2,5-difluorobenzènesulfonyl)-4-pipérazine-1-yl-1H-indole ;
5-chloro-1-(4-fluorobenzènesulfonyl)-4-pipérazine-1-yl-1H-indole ;
5-chloro-1-(4-chlorobenzènesulfonyl)-4-pipérazine-1-yl-1H-indole ;
5-chloro-1-(1,3,5-triméthyl-1H-pyrazole-4-sulfonyl)-4-pipérazine-1-yl-1H-indole ;
5-chloro-1-(pyridine-2-sulfonyl)-4-pipérazine-1-yl-1*H-*indole ;
5-chloro-1-(2,5-diméthylbenzènesulfonyl)-4-pipérazine-1-yl-1H-indole ;
3-chloro-1-(3-chlorobenzènesulfonyl)-4-pipérazine-1-yl-1H-indole ;
7-chloro-1-(3-chlorobenzènesulfonyl)-4-pipérazine-1-yl-1H-indole ;
7-chloro-1-(3,5-dichlorobenzènesulfonyl).-4-pipérazine-1-yl-1H-indole ;
1-benzènesulfonyl-7-chloro-4-pipérazine-1-yl-1*H*-indole ;
1-(4-bromo-2-trifluorométhoxybenzènesulfonyl)-7-chloro-4-pipérazine-1-yl-1H-indole ;
7-bromo-1-(3-chlorobenzènesulfonyl)-4-pipérazine-1-yl-1*H-*indole ;
7-cyano-1-(3-chlorobenzènesulfonyl)-4-pipérazine-1-yl-1*H-*indole ;
1-(3-chlorobenzènesulfonyl)-3,5-dichloro-4-pipérazine-1-yl-1H-indole ;
1-(3-chlorobenzènesulfonyl)-5,7-dichloro-4-pipérazine-1-yl-1H-indole ;
5-chloro-1-(3-chlorobenzènesulfonyl)-4-[1,4]diazépan-1-yl-1H-indole ;
5-chloro-1-(3-chlorobenzènesulfonyl)-4-((R)-3-méthylpipérazine-1-yl)-1H-indole ;
5-chloro-1-(3-chlorobenzènesulfonyl)-4-((S)-3-méthylpipérazine-1-yl)-1H-indole ;
1-(3-chlorobenzènesulfonyl)-5-cyano-4-pipérazine-1-yl-1H-indole ;
6-chloro-1-(3-chlorobenzènesulfonyl)-4-pipérazine-1-yl-1*H-*indole ;
5-chloro-1-(4-chloro-2-trifluorométhoxybenzènesulfonyl)-4-pipérazine-1-yl-1H-indole ;
1-(4-bromo-2-cyanobenzènesulfonyl)-5-chloro-4-pipérazine-1-yl-1H-indole ;
7-chloro-1-(3-chlorobenzènesulfonyl)-4-[1,4]diazépan-1-yl-1H-indole ;
ou un de ses sels pharmaceutiquement acceptables.

8. Composition pharmaceutique comprenant un composé de formule (I) répondant à la définition figurant dans l'une quelconque des revendications 1 à 7 ou un de ses sels pharmaceutiquement acceptables et un support ou excipient pharmaceutiquement acceptable.

9. Utilisation d'un composé de formule (I) répondant à la définition figurant dans l'une quelconque des revendications 1 à 7 dans la production d'un médicament destiné au traitement de la dépression, de l'anxiété, de troubles cognitifs de la mémoire, de la maladie d'Alzheimer, du déclin cognitif dû à l'âge, de l'altération cognitive faible, du syndrome de déficit d'attention/ hyperactivité et/ou de la schizophrénie.

10. Procédé pour la préparation d'un composé de formule (I) répondant à la définition figurant dans la revendication 1 ou d'un de ses sels pharmaceutiquement acceptables, procédé qui comprend
(a) le couplage d'un composé de formule (II) : 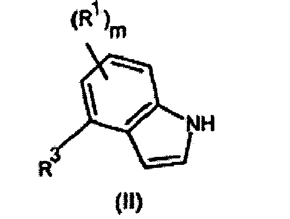 dans laquelle R¹, R³ et m répondent aux définitions mentionnées pour la formule (I) ou de ses dérivés protégés avec un composé de formule (III) 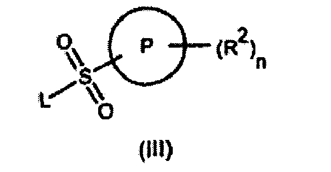 dans laquelle P, R² et n répondent aux définitions mentionnées pour la formule (I) ou un de ses dérivés protégés et L représente un groupe partant, et ensuite facultativement :
• l'élimination de tous les groupes protecteurs,
• la formation d'un sel pharmaceutiquement acceptable ;
(b) la préparation d'un composé de formule (I) dans laquelle R³ représente un groupe pipérazinyle ou 1,4-diazépanyle facultativement substitué lié au groupement indole par un atome d'azote, qui comprend la réaction d'un composé de formule (IV) 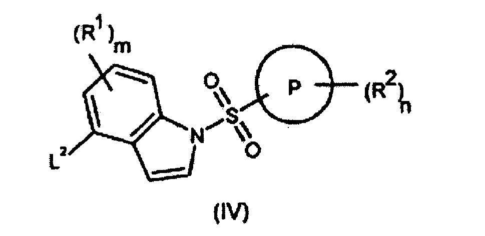 dans laquelle R¹, R² , P, m et n répondent aux définitions mentionnées pour la formule (I), ou d'un de ses dérivés protégés, et L² représente un groupe partant convenable (par exemple un atome d'halogène, tel qu'un atome de brome, ou un groupe trifluorométhylsulfonyloxy ou nonafluorobutylsulfonyloxy), avec un composé de formule R^{3'} -H, dans laquelle R³' représente un groupe pipérazinyle ou 1,4-diazépanyle facultativement protégé et/ou substitué, et ensuite, facultativement .
• l'élimination de tous les groupes protecteurs,
• la formation de sels pharmaceutiquement acceptables, ou
(c) la suppression de la protection d'un composé de formule (I) qui est protégé ; ou
(d) l' interconversion d'un composé de formule (I) en d'autres composés de formule (I).
